(19) Europäisches Patentamt
European Patent Office
Office européen des brevets

(11) **EP 2 832 288 A1**

# EUROPEAN PATENT APPLICATION

published in accordance with Art. 153(4) EPC

(43) Date of publication:
**04.02.2015 Bulletin 2015/06**

(21) Application number: **13768160.7**

(22) Date of filing: **26.03.2013**

(51) Int Cl.:
**A61B 5/0245** (2006.01) **A61B 5/11** (2006.01)

(86) International application number:
**PCT/JP2013/002066**

(87) International publication number:
**WO 2013/145731 (03.10.2013 Gazette 2013/40)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **30.03.2012 JP 2012080945**
**30.03.2012 JP 2012080946**

(71) Applicant: **Seiko Epson Corporation**
**Shinjuku-ku**
**Tokyo (JP)**

(72) Inventor: **TAKAHASHI, Yusuke**
**Suwa-shi**
**Nagano 392-8502 (JP)**

(74) Representative: **Hoffmann Eitle**
**Patent- und Rechtsanwälte PartmbB**
**Arabellastraße 30**
**81925 München (DE)**

(54) **PULSE DETECTION DEVICE, ELECTRONIC APPARATUS, AND PROGRAM**

(57)    To provide a pulsation detecting device, an electronic apparatus, a program, and the like that can perform frequency resolution processing with an improved real time property while maintaining resolution equivalent to the resolution in the method in the past.

A pulsation detecting device 100 includes a signal processing unit 130 that performs frequency resolution processing on the basis of a pulse wave detection signal from a pulse-wave detecting unit 210 and a body motion detection signal from a body-motion detecting unit 220, a pulsation-information calculating unit 170 that calculates pulsation information on the basis of a result of the frequency resolution processing, and a signal-state determining unit 150 that applies determination processing for a signal state to at least one of the pulse wave detection signal and the body motion detection signal. The signal processing unit 130 performs a different kind of the frequency resolution processing according to a determination result of the signal state by the signal-state determining unit 150.

FIG. 1

EP 2 832 288 A1

**Description**

Technical Field

The present invention relates to a pulsation detecting device, an electronic apparatus, a program, and the like.

Background Art

An electronic apparatus including a pulsation detecting device (a signal analyzing device) such as a pulse rate meter has been widely used. The pulsation detecting device is a device for detecting a pulsation deriving from a heartbeat of a human body and is a device for detecting a signal deriving from the heartbeat on the basis of signals received from pulse wave sensors attached to, for example, arms, palms, and fingers. When information concerning the signals deriving from the heartbeat (pulsation information) is actually presented to a user, for example, a pulse rate or the like is calculated as pulsation information and presented.

In calculating the pulsation information such as the pulse rate, main objects are two points: first, to calculate the pulsation information corresponding to as recent pulse wave detection signal as possible, and, second, to calculate more accurate pulsation information.

Concerning such objects, in the method disclosed in PTL 1, FFT (Fast Fourier Transform) processing is applied to a pulse wave detection signal in a predetermined period, a largest frequency component is determined as a pulse spectrum, and the frequency is converted into a pulse rate.

Citation List

Patent Literature

PTL 1: JP-A-2007-054471

Summary of Invention

Technical Problem

However, when a pulse rate is measured by such an approach, there are problems explained below. First, to more accurately calculate the pulse rate, the resolution of frequency resolution processing only has to be improved. However, to improve the resolution of the frequency resolution processing, it is necessary to extend a detection period of a pulse wave detection signal used for performing pulse rate calculation processing (pulsation information calculation processing) once. On the other hand, as the detection period of the pulse wave detection signal is further increased, a frequency component of a pulse wave detection signal further in the past is included in a frequency resolution processing result. That is, a real time property of the pulse rate is further deteriorated as the resolution of the frequency resolution processing is further improved.

That is, in the method in the past, it is difficult to attain both of the real time property of the pulse rate and the resolution of the frequency. A cause of this problem resides in an algorithm (a method) of the frequency resolution processing explained above rather than, for example, insufficiency of a processing ability of hardware.

According to several aspects of the present invention, it is possible to provide a pulsation detecting device, an electronic apparatus, a program, and the like that can perform frequency resolution processing with an improved real time property while maintaining resolution equivalent to the resolution in the method in the past.

Solution to Problem

An aspect of the present invention relates to a pulsation detecting device including: a signal processing unit that performs frequency resolution processing on the basis of a pulse wave detection signal from a pulse-wave detecting unit and a body motion detection signal from a body-motion detecting unit; a pulsation-information calculating unit that calculates pulsation information on the basis of a result of the frequency resolution processing; and a signal-state determining unit that applies determination processing for a signal state to at least one of the pulse wave detection signal and the body motion detection signal. The signal processing unit performs a different kind of the frequency resolution processing according to a determination result of the signal state by the signal-state determining unit.

In the aspect of the present invention, the determination processing for the signal state is applied to at least one of the pulse wave detection signal and the body motion detection signal. A different kind of the frequency resolution processing is performed according to a result of the determination processing to calculate the pulsation information.

Consequently, it is possible to, for example, calculate the pulsation information according to a method suitable for each of signal states.

In the aspect of the present invention, the signal processing unit may perform first frequency resolution processing when it is determined that the pulse wave detection signal is in a first signal state and perform second frequency resolution processing when it is determined that the pulse wave detection signal is in a second signal state in which an SN ratio (signal-noise ratio) is higher than an SN ratio in the first signal state.

Consequently, it is possible to, for example, perform a different kind of the frequency resolution processing according to the signal state in which the SN ratio is different.

In the aspect of the present invention, the signal processing unit may perform window function processing for sampling data of the pulse wave detection signal and Fourier transform processing for data after the window function processing as the first frequency resolution processing when it is determined that the pulse wave detection signal is in the first signal state and perform power spectral density estimation processing as the second frequency resolution processing when it is determined that the pulse wave detection signal is in the second signal state.

Consequently, it is possible to, for example, perform the frequency resolution processing with a high side lobe suppression effect when the pulse wave detection signal is in the first signal state in which the SN ratio is lower than the SN ratio in the second signal state and perform the frequency resolution processing with more importance placed on the real time property when the pulse wave detection signal is in the second signal state in which the SN ratio is higher than the SN ratio in the first signal state.

In the aspect of the present invention, jth sampling data $D_j$ among sampling data $D_0$ to $D_N$ (N is a positive integer equal to or larger than 2) of the pulse wave detection signal is data sampled at timing later than timing when ith sampling data $D_i$ is sampled (integers i and j are integers satisfying $0 \leq i < N/2 < j \leq N$). The signal processing unit may perform the window function processing using an asymmetrical window function, a window function value $h(j)$ of which corresponding to the jth sampling data $D_j$ is a maximum and a window function value $h(i)$ of which corresponding to the ith sampling data $D_i$ is a value smaller than the window function value $h(j)$, when it is determined that the pulse wave detection signal is in the first signal state.

Consequently, it is possible to, for example, improve a real time property of a frequency resolution processing result (a pulse rate) compared with when the window function processing is not performed or when the window function processing is performed using a symmetrical window function.

In the aspect of the present invention, the signal processing unit may perform window function processing for sampling data of the pulse wave detection signal, in which a first window function is used, and Fourier transform processing for first data after the window function processing as the first frequency resolution processing when it is determined that the pulse wave detection signal is in the first signal state and perform the window function processing for the sampling data of the pulse wave detection signal, in which a second window function different from the first window function is used, and the Fourier transform processing for second data after the window function processing as the second frequency resolution processing when it is determined that the pulse wave detection signal is in the second signal state.

Consequently, it is possible to, for example, perform a different kind of the window function processing according to the signal state.

In the aspect of the present invention, jth sampling data $D_j$ among sampling data $D_0$ to $D_N$ (N is a positive integer equal to or larger than 2) of the pulse wave detection signal is data sampled at timing later than timing when ith sampling data $D_i$ is sampled (integers i and j are integers satisfying $0 \leq i < N/2 < j \leq N$). The signal processing unit may perform the window function processing using an asymmetrical window function, a window function value $h(j)$ of which corresponding to the jth sampling data $D_j$ is a maximum and a window function value $h(i)$ of which corresponding to the ith sampling data $D_i$ is a value smaller than the window function value $h(j)$, as the first window function when it is determined that the pulse wave detection signal is in the first signal state and perform the window function processing using a second window function, which is the asymmetrical window function different from the first window function, when it is determined that the pulse wave detection signal is in the second signal state.

Consequently, it is possible to, for example, change the asymmetrical window function used in the window function processing according to the signal state.

In the aspect of the present invention, the signal-state determining unit may calculate a ratio of spectrum values of a first spectrum indicating a maximum spectrum value and at least one second spectrum other than the first spectrum among frequency spectra obtained by the frequency resolution processing of the pulse wave detection signal and perform the determination processing for the signal state.

Consequently, it is possible to, for example, estimate an SN ratio and calculate a signal state.

In the aspect of the present invention, the signal-state determining unit may perform processing for determining an exercise state of a subject on the basis of the body motion detection signal as the determination processing for the signal state. The signal processing unit may perform a different kind of the frequency resolution processing according to the exercise state.

Consequently, it is possible to, for example, perform a different kind of the frequency resolution processing according to the exercise state.

In the aspect of the present invention, the signal processing unit may perform first frequency resolution processing when it is determined that the exercise state is a steady exercise state and perform second frequency resolution processing when it is determined that the exercise state is an unsteady exercise state.

Consequently, it is possible to, for example, perform different kinds of the frequency resolution processing when the pulse wave detection signal has periodicity and when the pulse wave detection signal does not have periodicity.

In the aspect of the present invention, the signal processing unit may perform window function processing for sampling data of the pulse wave detection signal, in which a first window function is used, and Fourier transform processing for first data after the window function processing as the first frequency resolution processing when it is determined that the exercise state is the steady exercise state and perform the window function processing for the sampling data of the pulse wave detection signal, in which a second window function different from the first window function is used, and the Fourier transform processing for second data after the window function processing as the second frequency resolution processing when it is determined that the exercise state is the unsteady exercise state.

Consequently, it is possible to, for example, perform the window function processing respectively using the different window functions when the pulse wave detection signal has periodicity and when the pulse wave detection signal does not have periodicity.

In the aspect of the present invention, j th sampling data $D_j$ among sampling data $D_0$ to $D_N$ (N is a positive integer equal to or larger than 2) of the pulse wave detection signal is data sampled at timing later than timing when ith sampling data $D_i$ is sampled (integers i andj are integers satisfying $0 \leq i < N/2 < j \leq N$). The signal processing unit may perform the window function processing using an asymmetrical window function, a window function value $h(j)$ of which corresponding to the jth sampling data $D_j$ is a maximum and a window function value $h(i)$ of which corresponding to the ith sampling data $D_i$ is a value smaller than the window function value $h(j)$, as the first window function when it is determined that the exercise state is the steady exercise state and perform the window function processing using a second window function, which is the asymmetrical window function different from the first window function, when it is determined that the exercise state is the unsteady exercise state.

Consequently, it is possible to, for example, perform the window function processing respectively using the different asymmetrical window functions when the pulse wave detection signal has periodicity and when the pulse wave detection signal does not have periodicity.

In the aspect of the present invention, the signal processing unit may perform first frequency resolution processing when it is determined that the exercise state is a rest state and perform second frequency resolution processing when it is determined that the exercise state is a steady exercise state or an unsteady exercise state.

Consequently, it is possible to, for example, perform different kinds of the frequency resolution processing when the exercise state is the rest state and when the exercise state is the steady exercise state or the unsteady exercise state.

In the aspect of the present invention, the signal processing unit may perform power spectral density estimation processing as the first frequency resolution processing when it is determined that the exercise state is the rest state and perform window function processing for sampling data of the pulse wave detection signal and Fourier transform processing for data after the window function processing as the second frequency resolution processing when it is determined that the exercise state is the in-exercise state.

Consequently, it is possible to, for example, perform frequency resolution processing with a high suppression effect of a side lobe when the exercise state is the steady exercise state or the unsteady exercise state and perform frequency resolution processing with more importance placed on a real time property when the exercise state is the rest state.

In the aspect of the present invention, jth sampling data $D_j$ among sampling data $D_0$ to $D_N$ (N is a positive integer equal to or larger than 2) of the pulse wave detection signal is data sampled at timing later than timing when ith sampling data $D_i$ is sampled (integers i and j are integers satisfying $0 \leq i < N/2 < j \leq N$). The signal processing unit may perform the window function processing using an asymmetrical window function, a window function value $h(j)$ of which corresponding to the jth sampling data $D_j$ is a maximum and a window function value $h(i)$ of which corresponding to the ith sampling data $D_i$ is a value smaller than the window function value $h(j)$, as the first window function when it is determined that the exercise state is the steady exercise state or the unsteady exercise state.

Consequently, it is possible to, for example, perform the window function processing by the asymmetrical window function when the exercise state is the steady exercise state or the unsteady exercise state and perform the frequency resolution processing with an improved real time property.

Another aspect of the present invention relates to an electronic apparatus including the signal analyzing device.

Another aspect of the present invention relates to a program for causing a computer to function as the units.

Brief Description of Drawings

[Fig. 1] Fig. 1 is a system configuration diagram in an embodiment.

[Fig. 2] Fig. 2(A) and Fig. 2(B) are examples of an electronic apparatus including a pulsation detecting device.

[Fig. 3] Fig. 3 is a comparison diagram of shapes of window functions.

[Fig. 4] Fig. 4 is a comparison diagram of characteristics of kinds of frequency resolution processing.

[Fig. 5] Fig. 5 is a flowchart for explaining a flow of overall processing in a first embodiment.

[Fig. 6] Fig. 6 is a flowchart for explaining a flow of determination processing for a signal state in the first embodiment.

[Fig. 7] Fig. 7(A) to Fig. 7(C) are explanatory diagrams of a case in which it is determined that a signal state is good.

[Fig. 8] Fig. 8 (A) to Fig. 8(C) are explanatory diagrams of a case in which a signal state is medium.

[Fig. 9] Fig. 9 (A) to Fig. 9(C) are explanatory diagrams of a case in which a signal state is bad.

[Fig. 10] Fig. 10 (A) to Fig. 10 (C) are comparison diagrams of window function processing results.

[Fig. 11] Fig. 11 is a flowchart for explaining a flow of overall processing in a second embodiment.

[Fig. 12] Fig. 12 is a flowchart for explaining a flow of determination processing for a signal state in the second embodiment.

[Fig. 13] Fig. 13 is a comparison diagram of measurement results of a pulse rate.

[Fig. 14] Fig. 14 (A) to Fig. 14 (C) are comparison diagrams of spectra of acceleration detection signals in exercise states.

[Fig. 15] Fig. 15 (A) and Fig. 15 (B) are specific examples showing spectra of acceleration detection signals during an exercise start.

[Fig. 16] Fig. 16 (A) and Fig. 16 (B) are specific examples showing spectra of acceleration detection signals during an exercise stop.

Description of Embodiments

Embodiments are explained below. First, an overview of a method in this embodiment is explained. Then, a system configuration example is explained. A first embodiment and a second embodiment are explained using flowcharts and the like. Lastly, other application examples of the first embodiment and the second embodiment are explained. Note that the embodiments explained below do not unduly limit contents of the present invention described in claims. Not all components explained in the embodiments are always essential constituent elements of the present invention.

1. Overview

An electronic apparatus including a pulsation detecting device such as a pulse rate meter is widely used. The pulsation detecting device is a device for detecting a pulsation deriving from a heartbeat of a human body and is a device for detecting a signal deriving from the heartbeat on the basis of signals received from pulse wave sensors attached to, for example, arms, palms, and fingers. When information concerning the signals deriving from the heartbeat (pulsation information) is actually presented to a user, for example, a pulse rate or the like is calculated as pulsation information and presented.

In calculating the pulsation information such as the pulse rate, main objects are two issues: first, to calculate the pulsation information corresponding to as recent pulse wave detection signal as possible, and, second, to calculate more accurate pulsation information.

Concerning such objects, in the method disclosed in PTL 1, FFT (Fast Fourier Transform) processing is applied to a pulse wave detection signal in a predetermined period, a largest frequency component is determined as a pulse spectrum, and the frequency is converted into a pulse rate.

However, when a pulse rate is measured by such an approach, there are problems explained below. First, to more accurately calculate the pulse rate, the resolution of frequency resolution processing only has to be improved. However, to improve the resolution of the frequency resolution processing, it is necessary to extend a detection period of a pulse wave detection signal used for performing pulse rate calculation processing (pulsation information calculation processing) once.

On the other hand, when the detection period of the pulse wave detection signal is extended, a required real time property is sometimes not satisfied.

The real time property means that a worst value of a response time is guaranteed, that is, a response is returned within a requested response time. The real time property is not a concept indicating that a response is quick or late. Note that the requested response time is different for each of systems. Therefore, for example, when a response time (a response period) required of a certain system is 32 days, even if the system returns a response in a full one month, the system is considered to satisfy the real time property.

Required strictness of the real time property is different depending on a system. As the real time property, there is a hard real time property for not allowing even a slight delay and a soft real time property for not regarding a slight delay as a problem. For example, for processing performed by firmware for causing hardware to operate, the hard real time property for not allowing even a delay in microsecond units is often required. On the other hand, for a weather forecast or the like, the soft real time property for allowing a delay of about several minutes is required. These classifications represent tolerance for a response delay and are unrelated to speed of a response.

The real time property required of the pulsation detecting device is the soft real time property. However, it is desirable that a required response time is several second units and the pulsation detecting device can detect a pulse rate or the like corresponding to timing as close as possible to the present.

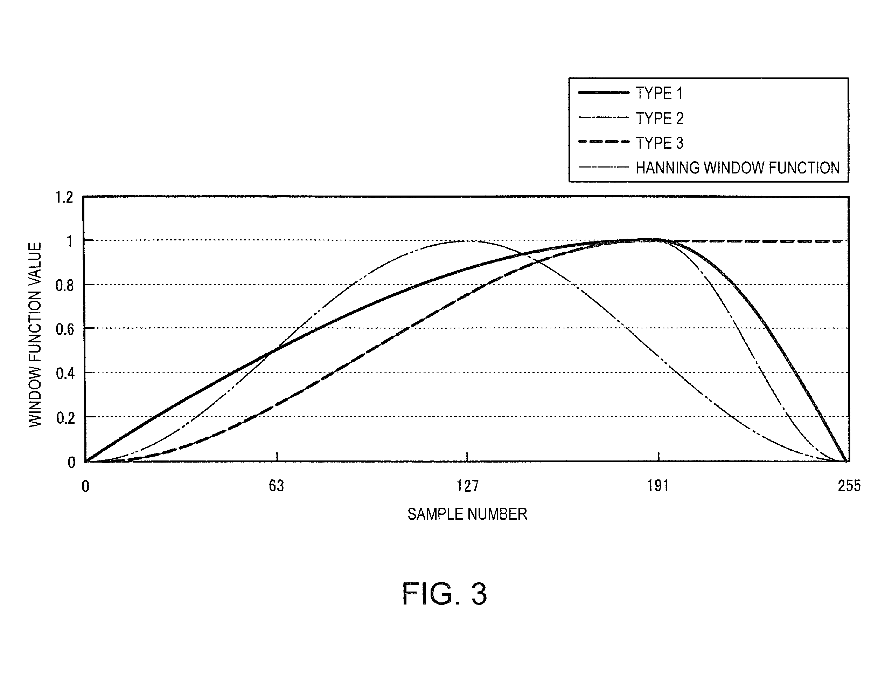

As explained above, in calculating the pulse rate or the like, frequency resolution processing is applied to a pulse wave detection signal in a predetermined period. In the frequency resolution processing, after window function processing is applied to the pulse wave detection signal using a Hanning window or the like shown in Fig. 3 explained below, FFT processing is applied to the pulse wave detection signal. In a window function such as the Hanning window widely used in general, a window function value is maximized in the middle (the middle point) of a period of an input signal. Therefore, a spectral distribution drawn as a result of the frequency resolution processing is a distribution indicating an expected value in the past by a half time of the period of the input signal. Therefore, a pulse rate calculated on the basis of the spectral distribution is also calculated as an expected value in the past by the half time of the period of the input signal. For example, when a detection period of the pulse wave detection signal is 4 seconds, a pulse rate before 2 seconds is calculated as the expected value. When the detection period of the pulse wave detection signal is 16 seconds, a pulse rate before 8 seconds is calculated as the expected value.

In this way, resolution is improved when the detection period of the pulse wave detection signal input to the frequency resolution processing is extended. However, an expected value-like spectral distribution at a point further in the past is calculated. The real time property is deteriorated. A cause of such deterioration in the real time property resides in an algorithm (a method) of the frequency resolution processing explained above rather than, for example, insufficiency of a processing ability of hardware.

Therefore, the applicant proposes a new window function explained in detail below. A signal analyzing device or the like that uses the window function proposed by the applicant can perform frequency resolution processing with an improved real time property while maintaining resolution equivalent to the resolution in the method in the past.

2. System configuration example

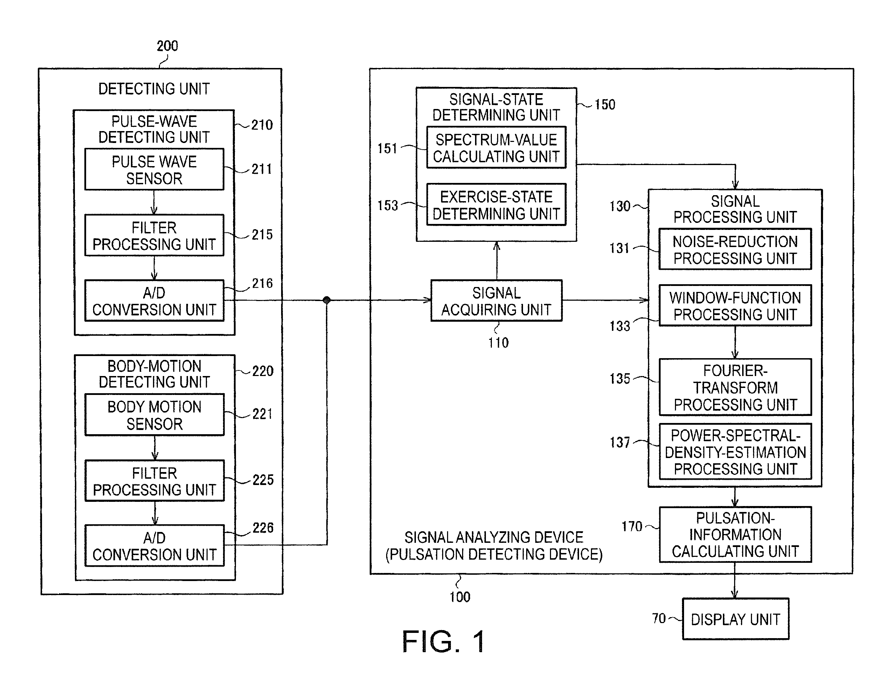

A configuration example of a signal analyzing device (a pulsation detecting device) 100 and an electronic apparatus including the signal analyzing device (the pulsation detecting device) 100 in a first embodiment and a second embodiment explained blow is shown in Fig. 1.

The signal analyzing device (the pulsation detecting device) 100 includes a signal acquiring unit 110, a signal processing unit 130, and a signal-state determining unit 150. Examples of the electronic apparatus including the signal analyzing device (the pulsation detecting device) 100 include a portable terminal including a detecting unit 200 and a display unit 70. Note that the signal analyzing device (the pulsation detecting device) 100 and the electronic apparatus including the signal analyzing device (the pulsation detecting device) 100 is not limited to the configuration shown in Fig. 1. Various modifications are possible, for example, a part of components thereof are omitted and other components are added.

To more specifically describe the operations in the first embodiment and the second embodiment, as an example, the signal analyzing device 100 is used as the pulsation detecting device 100. However, the present invention and the scope of the present invention are not limited to the pulsation detecting device 100. The signal analyzing device 100 can also be used for other uses as explained below.

Processing performed by the units is explained. First, the detecting unit 200 is explained and a signal and the like input to the signal analyzing device (the pulsation detecting device) 100 are explained. Then, the units of the signal analyzing device 100 are explained.

First, the detecting unit 200 includes a pulse-wave detecting unit 210 and a body-motion detecting unit 220. Note that the detecting unit 200 is not limited to the configuration shown in Fig. 1. Various modifications are possible, for example, a part of components thereof are omitted and other components are added.

The pulse-wave detecting unit 210 outputs a pulse wave detection signal on the basis of sensor information (a pulse wave sensor signal) obtained from a pulse wave sensor 211. The pulse-wave detecting unit 210 can include, for example, the pulse wave sensor 211, a filter processing unit 215, and an A/D conversion unit 216. However, the pulse-wave detecting unit 210 is not limited to the configuration shown in Fig. 1. Various modifications are possible, for example, a part of components thereof are omitted and other components, for example, an amplifying unit that amplifies a signal are added.

The pulse wave sensor 211 is a sensor for detecting a pulse wave sensor signal. For example, a photoelectric sensor is conceivable. Note that, when the photoelectric sensor is used as the pulse wave sensor 211, a sensor configured to cut a signal component of external light such as sunlight may be used. This can be realized by, for example, a configuration in which a plurality of photodiodes are provided and difference information is calculated by feedback processing or the like using signals of the photodiodes.

Note that the pulse wave sensor 211 is not limited to the photoelectric sensor and may be a sensor that uses ultrasound. In this case, the pulse wave sensor 211 includes two piezoelectric elements. The pulse wave sensor 211 excites one piezoelectric element to transmit ultrasound to the inside of an organism and receives, with the other piezoelectric element, the ultrasound reflected by a blood flow of the organism. In the transmitted ultrasound and the received ultrasound, a frequency change is caused by a Doppler effect of the blood flow. Therefore, in this case, it is possible to acquire a signal corresponding to a flood flow amount. It is possible to estimate pulsation information. Other sensors may be used as the pulse wave sensor 211.

The filter processing unit 215 applies high-pass filter processing to the pulse wave sensor signal from the pulse wave sensor 211. Note that a cutoff frequency of the high-pass filter may be calculated from a typical pulse rate. For example, a pulse rate of a normal person is extremely rarely lower than thirty per minute. That is, the frequency of a signal deriving from a heartbeat is rarely equal to or lower than 0.5 Hz. Therefore, even if information concerning a frequency band in this range is cut, an adverse effect on a signal desired to be acquired is small. Therefore, about 0.5 Hz may be set as the cutoff frequency. A different cutoff frequency such as 1 Hz may be set depending on a situation. Further, since a typical upper limit value can be assumed for a pulse rate of a person, the filter processing unit 215 may perform band-pass filter processing rather than the high-pass filter processing. A cutoff frequency on a high frequency side can also be set freely to a certain degree. However, a value such as 4 Hz only has to be used.

The A/D conversion unit 216 performs A/D conversion processing and outputs a digital signal. Note that the processing in the filter processing unit 215 maybe analog filter processing performed before the A/D conversion processing or may be digital filter processing performed after the A/D conversion processing.

The body-motion detecting unit 220 outputs a signal corresponding to a body motion (a body motion detection signal) on the basis of sensor information (body motion sensor signals) of various sensors. The body-motion detecting unit 220 can include, for example, a body motion sensor 221, a filter processing unit 225, and an A/D conversion unit 226. However, the body-motion detecting unit 220 may include other sensors (e.g., a gyro sensor) and an amplifying unit that amplifies a signal.

As the body motion sensor, amotionsensor (anacceleration sensor), a pressure sensor (a contact pressure sensor), or the like is used. As the body motion sensor, a plurality of kinds of sensors may be provided. The body motion sensor may include one kind of sensor.

The motion sensor is, for example, an acceleration sensor. The acceleration sensor is configuredby, for example, anelement, a resistance value of which increases and decreases according to an external force. The motion sensor detects acceleration information of three axes.

The pressure sensor is, for example, a contact pressure sensor. The contact pressure sensor may come into direct contact with a subject and measure a contact pressure or may indirectly measure a contact pressure using a cuff structure or the like. That is, the contact pressure sensor may be a sensor including a piezoelectric element or may be an atmospheric pressure sensor or the like.

The filter processing unit 225 applies various kinds of filter processing to a body motion sensor signal. For example, the filter processing unit 225 performs high-pass filter processing or performs band-pass filter processing or the like. However, filter processing does not always have to be performed. The filter processing unit 225 does not have to be included.

Processing performed by the A/D conversion unit 226 is substantially the same as the processing of the A/D conversion unit 216. Therefore, explanation of the processing is omitted.

Terms are explained here. First, a pulsation means an expanding and contracting motion of peripheral blood vessels. A pulse wave is obtained by grasping, as a signal, a capacity change caused by inflow of blood into a body tissue. For example, the pulse wave is obtained by grasping, as a signal waveform, an LED irradiated on a body surface from a pulse wave sensor and capturing scattered light and reflected light of the LED with a photodiode. The pulse wave is obtained by grasping blood vessel motion reaction rather than the motion itself of the heart. The pulse wave includes noise-like factors other than the motion of the heart, for example, a capacity change of blood vessels caused by exercise, action, and the like of a human. To correctly grasp the motion itself of the heart and a pulse rate, it is necessary to eliminate the noise-like factor.

On the other hand, in a medical sense, the pulsation indicates a motion caused when cyclic contraction and relaxation of not only the heart but also internal organs in general are repeated. In particular, a motion of the heart functioning as a pump for cyclically feeding blood is referred to as pulsation.

Further, the body motion means movement of a body in a broad sense. In a narrow sense, the body motion indicates steady and cyclic arm (near a wearing part of a pulse rate meter) motion or the like involved in walking, jogging, or the like.

The pulse wave sensor signal (a pulse wave sensor original signal and a pulse wave signal) means a signal itself detected by the pulse wave sensor 211. The pulse wave sensor signal includes a pulsation component signal, a body motion noise component signal, and a disturbance noise component signal. Note that an irregular pulse is considered to be included in the pulsation component signal in a strict sense. However, the irregular pulse is included in a disturbance noise component signal as an electric signal.

On the other hand, the pulse wave detection signal means a signal output from the pulse-wave detecting unit 210. Specifically, the pulse wave detection signal means, for example, a signal obtained by applying the filter processing to the pulse wave sensor signal with the filter processing unit 215 and applying the A/D conversion processing to the pulse wave sensor signal after the filter processing with the A/D conversion unit 216. However, as explained above, the order of the filter processing and the A/D conversion processing may be opposite.

Further, the body motion sensor signal (a body motion sensor original signal and a body motion signal) means a signal itself detected by the body motion sensor. Specifically, the body motion sensor signal indicates, for example, a motion sensor signal indicating a signal itself detected by the motion sensor 221 or a contact pressure sensor signal indicating a signal itself detected by the contact pressure sensor.

On the other hand, the body motion detection signal means a signal output from the body-motion detecting unit 220. Specifically, the body motion detection signal means, for example, a signal after the filter processing and the A/D conversion processing are applied to the body motion sensor signal. For example, the body motion detection signal means a motion detection signal, which is a signal after the signal processing is applied to the motion sensor signal, or a pressure detection signal, which is a signal after the signal processing is applied to the pressure sensor signal. However, as explained above, the order of the filter processing and the A/D conversion processing may be opposite.

The pulse wave sensor signal, the bodymotion sensor signal, and the like are collectively referred to as sensor signal. The pulse wave detection signal, the body motion detection signal, and the like are collectively referred to as detection signal.

Processing performed in the units of the signal analyzing device (the pulsation detecting device) 100 is explained.

First, the signal acquiring unit 110 acquires a detection signal from the detecting unit 200. The signal acquiring unit 110 of the pulsation detecting device 100 acquires a pulse wave detection signal from the pulse-wave detecting unit 210 and a body motion detection signal from the body-motion detecting unit 220. When the signal acquiring unit 110 is connected to the detecting unit 200 by radio or wire, the signal acquiring unit 110 may be a communication unit (an I/F unit, an antenna unit, etc.) that transmits and receives data.

The signal processing unit 130 applies frequency resolution processing to sampling data of a detection signal and calculates a spectrum. For example, as the frequency resolution processing, the signal processing unit 130 performs window functionprocessing for the sampling data of the detection signal and Fourier transform processing for data after the window function processing. The signal processing unit 130 of the pulsation detecting device 100 performs the frequency resolution processing on the basis of the pulsation detection signal from the pulse-wave detecting unit 210 and the body motion detection signal from the body-motion detecting unit 220 and calculates a pulse spectrum.

The signal processing unit 130 can include a noise-reduction processing unit 131, a window-function processing unit 133, a Fourier-transform processing unit 135, and a power-spectral-density-estimation processing unit 137. Details of the operations of these processing units are explained below.

The signal-state determining unit 150 applies determination processing for a signal state to the detection signal acquired by the signal acquiring unit 110. The signal-state determining unit 150 of the pulsation detecting device 100 applies determinationprocessing for the signal state to at least one of the pulse wave detection signal and the body motion detection signal.

The signal-state determining unit 150 can include a spectrum-value calculating unit 151 and an exercise-state determining unit 153. Details of the operations of these processing units are explained below.

Further, when the signal analyzing device 100 is the pulsation detecting device 100, as shown in Fig. 1, the signal analyzing device 100 can include a pulsation-information calculating unit 170. The pulsation-information calculating unit 170 calculates pulsation information on the basis of a result of the frequency resolution processing calculated by the signal processing unit 130. The pulsation information means information obtained by representing a pulsation with a numerical value or the like and indicates, for example, a pulse rate.

For example, the pulsation-information calculating unit 170 performs processing for converting an analysis result (a frequency value of a pulse) of the signal processing unit 130 into a pulse rate while applying smoothing processing or the like to the analysis result. In calculating the pulse rate, the pulsation-information calculating unit 170 may perform processing for setting a representative frequency as a pulse frequency in the pulse spectrum calculated by the signal processing unit 130. In that case, a value obtained by multiplying the calculated pulse frequency by 60 is a pulse rate (a heartbeat rate) used in general.

Note that the pulsation information is not limited to the pulse rate and may be, for example other information (the frequency, the cycle, and the like of the heartbeat) representing the pulse rate. The pulsation information may be information representing a state of a pulsation. For example, a value representing a blood flow amount itself (or fluctuation in the blood flow amount) may be the pulsation information. However, there is an individual difference of each user in a relation between the flood flow amount and the signal value of the pulse wave sensor signal. Therefore, it is desirable to perform correction processing for coping with the individual difference when the blood flow amount or the like is used as the pulsation information.

When the signal analyzing device 100 is not the pulsation detecting device 100, the signal analyzing device 100 may include, instead of the pulsation-information calculating unit 170, a processing unit that calculates other information.

The display unit 70 (an output unit in a broad sense) is a unit for displaying various display screens used for presenting the calculated pulsation information and the like. The display unit 70 can be realized by, for example, a liquid crystal display and an organic EL display.

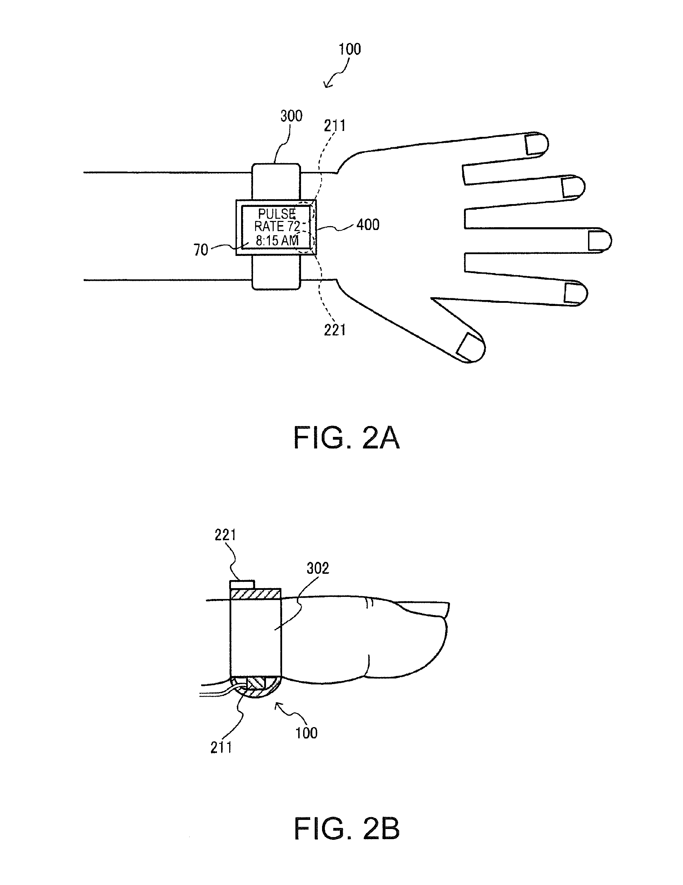

Specific examples of the electronic apparatus functioning as a pulse rate meter are shown in Fig. 2(A) and Fig. 2(B). Fig. 2 (A) is an example of a pulse rate meter of a watch type. A base unit 400 including the pulse wave sensor 211 and the display unit 70 is mounted on the left wrist of a subject (a user) by a retaining mechanism 300 (e.g., a band). Fig. 2 (B) is an example of a finger mounted type. The pulse wave sensor 211 is provided in the bottom of a ring-like guide 302 for putting the pulse rate meter on a fingertip of the subject. However, in the case of Fig. 2(B), since there is no spatial room for providing the display unit 70, it is assumed that the display unit 70 (and, when necessary, a device equivalent to the pulsation detecting device 100) is separately provided. However, the electronic apparatus including the signal analyzing device (the pulsation detecting device) 100 is not limited to the pulse rate meter and may be a pedometer, a smart phone, or the like.

3. A symmetrical window function

A window function used in the signal analyzing device (the pulsation detecting device) 100 in the first embodiment and the second embodiment is explained with reference to Fig. 3. Fig. 3 is a graph in which shapes of window functions are compared when the number of samples is 256. The ordinate indicates a widow function value and the ordinate indicates a sample number. The window function value is a weight to be multiplied with the samples.

First, the Hanning window function has a symmetrical shape on the graph shown in Fig. 3. Besides the Hanning window function, a window function used in general such as a Blackman window function has the same shape. In this way, in general, the window function having the symmetrical shape on the graph shown in Fig. 3 (hereinafter referred to as symmetrical window function) is used to improve a side lobe characteristic and more clearly show a difference in the magnitude of power between power spectra adjacent to each other. Note that the side lobe characteristic means a degree of detection of amplitude of a frequency other than a main component.

In the first place, the Fourier transform means representing a signal in any time domain in a frequency domain on the major premise that all periodic functions can be represented by superimposition of a sine wave and a cosine wave. That is, as a premise, it is important that the Fourier transform is applied to a periodic function.

In a spectrum obtained when a completely cyclic time signal is subjected to the Fourier transform, only a single frequency is detected. That is, the side lobe characteristic is good and it is easy to detect a main component. On the other hand, in a spectrum obtained when a non-cyclic time signal is subj ected to the Fourier transform, apluralityof frequencies are detected in a wide range of a low frequency to a high frequency. That is, the side lobe characteristic is bad and it is difficult to determine which frequency the main component is. The purpose of performing the Fourier transform is often detection of a representative frequency (the main component) of the time signal. Therefore, it is preferable that the side lobe characteristic is good.

However, when the Fourier transform is actually performed, an unlimited number of samples cannot be taken from a certain time signal. Therefore, a limited number of samples are taken from a limited range (period) of a certain time signal as sampling data and the sampling data is used as an input signal. In particular, when FFT is performed, there is a limitation that the number of samples of the input signal has to be the power of 2 such as 64, 128, or 256. However, possibility that the sampling data including the limited number of samples can be represented by a periodic function is extremely low. Moreover, a frequency often changes with time.

Further, when discrete Fourier transform is performed, if continuity of a start point and an end point of the sampling data including the limited number of samples are not secured, a side lobe appearing in a frequency resolution result is large.

Therefore, the window function processing is applied to the sampling data to secure the continuity of the sampling data including the limited number of samples. As a method of enabling the sampling data to be represented by the periodic function when the sampling data is repeated, there are several methods. As a most simple method, there is a method of setting values at both ends of the sampling data to 0 and setting gradients at both the ends to 0. In this case, the sampling data can be represented by a periodic function for setting a sampling period to one cycle. A window function such as a Hanning window adopts such an approach. Therefore, the window function has the shape shown in the graph of Fig. 3.

Frequency resolution processing for performing the window function processing using such a window function before the Fourier transform processing is performed in, for example, the field of communication. In that case, for example, a signal is received by radio communication or the like, the frequency resolution processing or the like is performed, and original information converted into a signal is analyzed. Therefore, if a waveform of a signal transmitted by a transmission source is substantially changed, original information converted into the signal sometimes cannot be analyzed. Therefore, in the window function processing, it is necessary to maintain a waveform of an original signal as much as possible while processing the signal into a cyclic signal. Therefore, the widow function such as the Hanning window is designed to be symmetrical in the graph of Fig. 3.

However, when the frequency resolution processing is performed using the symmetrical window function in the field of pulsation detection (measurement), there is a problem in that the real time property is deteriorated when the frequency resolutionisincreased asexplained above. This problem occurs because a window function value is maximized in the middle of a sampling period in the target window function such as the Hanning window. That is, as the sampling period is further extended, timing when the window function value is maximized is further in the past. As a requested response time is shorter, it is more difficult to satisfy the real time property.

On the other hand, information desired to be acquired by a part of signal analyzing devices such as a pulsation detecting device is considered to be a main frequency (a main component) of a time signal. That is, a waveform of a signal (the amplitude of the signal) itself is not important. The shape of the waveform may be deformed. The main component of the frequency of the time signal is important. Specifically, in the pulsation detecting device, a pulse rate indicating how many times the heart beats in 1 minute is desired to be known. However, information indicating how strong the heart beats is not always necessary.

Therefore, the applicant devised a window function in which timing when the window function value is maximized is intentionally shifted from the middle of the sampling period. As it is seen from the example explained above, a frequency detected as a result of the frequency resolution processing has a strong correlation with an actual frequency at timing of a maximum window function value and is considered to be an expected value of a frequency at the timing of the maximum window function value.

Therefore, a window function in which the timing of the maximum window function value is shifted to the present timing from the middle of the sampling period is used. Consequently, weight is further increased with respect to a latest sample and a signal level is compressed with respect to a sample in the past. Consequently, compared with when the window function processing is not performed or when the window function processing is performed using the symmetrical window function, the real time property of a frequency resolution processing result (a pulse rate) is improved.

As a specific example, in a window function having an asymmetrical shape (an asymmetrical window function), for example, window functions such as Type 1 to Type 3 shown in Fig. 3 are proposed.

The window functions of Type 1 to Type 3 are based on a triangular function. The window function is configured by a frequency band equal to or lower than 0.5 Hz. The frequency of the window function itself deviates from a frequency band of a pulse that could be taken in a normal human body. Therefore, in a calculate spectrum, a spectrum of the pulse (equal to or higher than 0.5 Hz) and a spectrum of the window function are not confused.

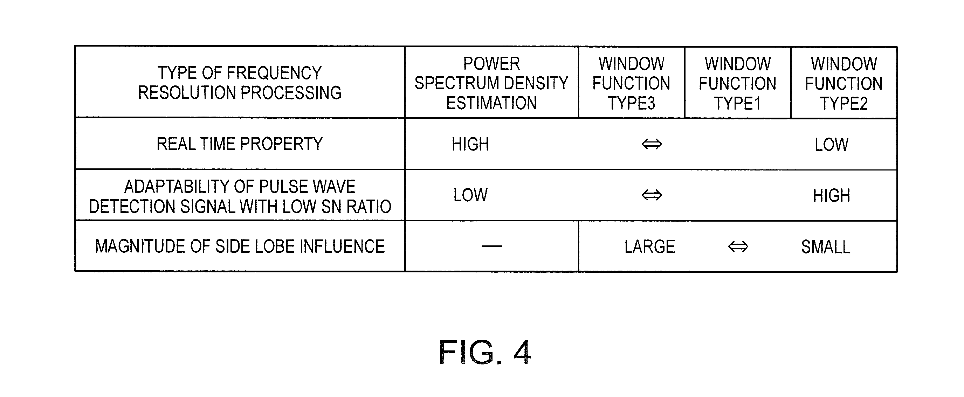

In the window function of Type 3, compression of a signal level is not performed at all with respect to a latest signal sample. As shown in a table of Fig. 4 in which characteristics of kinds of frequency resolution processing are compared, compared with when values at both ends of an FFT section (a sampling section) are set to 0 and gradients at both ends is set to 0 (the Hanning window function or the window function of Type 2 explained blow), the side lobe characteristic is sacrificed and adaptability to a case in which a pulse wave detection signal includes a lot of noise is deteriorated. However, on the other hand, it is possible to further improve the real time property.

In the window function of Type 2, a signal level is compressed to set values at both ends of an input signal to 0 and set gradients at both the ends to 0. As shown in Fig. 4, the influence of the shift of the timing when the widow function value is maximized is not zero. However, the window function of Type 2 has a satisfactory side lobe characteristic compared with the window function of Type 3 and the like. Therefore, the window function of Type 2 is suitably used when the pulse wave detection signal includes a lot of noise.

In the window function of Type 1, the signal level is compressed to set the values at both the ends of the input signal to 0. However, since the gradients at both the ends are not set to 0, as shown in Fig. 4, the performance of the side lobe characteristic is slightly low compare with Type 2. On the other hand, since a compression degree of a signal is small compared with Type 2, even when a level of the pulse wave detection signal is low, a power spectrum value indicating pulsation tends to be clearer. Note that specific expressions of the window functions of Type 1 to Type 3 are explained below.

4. First Embodiment

4. 1. Method

A method in a first embodiment is explained. The signal analyzing device (the pulsation detecting device) 100 in the first embodiment selects a procedure of frequency resolution processing according to a state of an SN ratio (signal-noise ratio) of a pulse wave detection signal.

First of all, when it is determined that a state of the SN ratio is satisfactory, that is, when it is determined that body motion noise and mixing of sudden disturbance are little, the pulsation detecting device 100 performs power spectral density estimating processing as the frequency resolution processing. In this case, the pulsation detecting device 100 does not perform the window function processing using the asymmetrical window function. This is because the frequency resolution processing with sufficiently high accuracy is considered to be capable of being performed even if the window function processing is not performed. As a specific method of the power spectral density estimation processing, there are an AR (Auto-Regressive) method, a maximum entropy method, and the like.

Consequently, it is possible to obtain a frequency distribution on the basis of a pulse wave detection signal waveform immediately before observation timing. Therefore, it is possible to further improve the real time property.

Second, when it is determined that the state of the SN ratio is inferior, that is, for example, when a ratio of the body motion noise is high or when mixing of sudden and large disturbance is detected, as the frequency resolutionprocessing, the pulsation detecting device 100 performs the window function processing using the asymmetrical window function of Type 1 or Type 2. The FFT is applied to data after the window function processing.

Consequently, compared with when window function processing is not performed or when the window function processing is performed using a general window function, the real time property of a frequency resolution processing result (a pulse rate) is improved. Further, compared with when the window function processing is performed using the window function of Type 3 explained below, it is possible to further suppress the side lobe and improve the side lobe characteristic. Therefore, even in a pulsation waveform having very small amplitude, it is easy to specify a pulsation spectrum.

Third, when it is determined that the state of the SN ratio is in the middle between the satisfactory case and the inferior case, as the frequency resolution processing, the window function processing is performed using the asymmetrical window function of Type 3 explained above and the FFT is applied to data after the window function processing.

Consequently, compared with not only when the window function processing is not performed but also when the window function processing is performed using the asymmetrical window function of Type 1 or Type 2, the real time property of the frequency resolution processing result (the pulse rate) is improved. On the other hand, a suppression effect of the side lobe is lower than when the asymmetrical window function of Type 1 or Type 2 is used. However, in the first place, since the window function processing is applied when the state of the SN ratio is not so bad, the suppression effect is not a problem.

4.2. Flow of processing

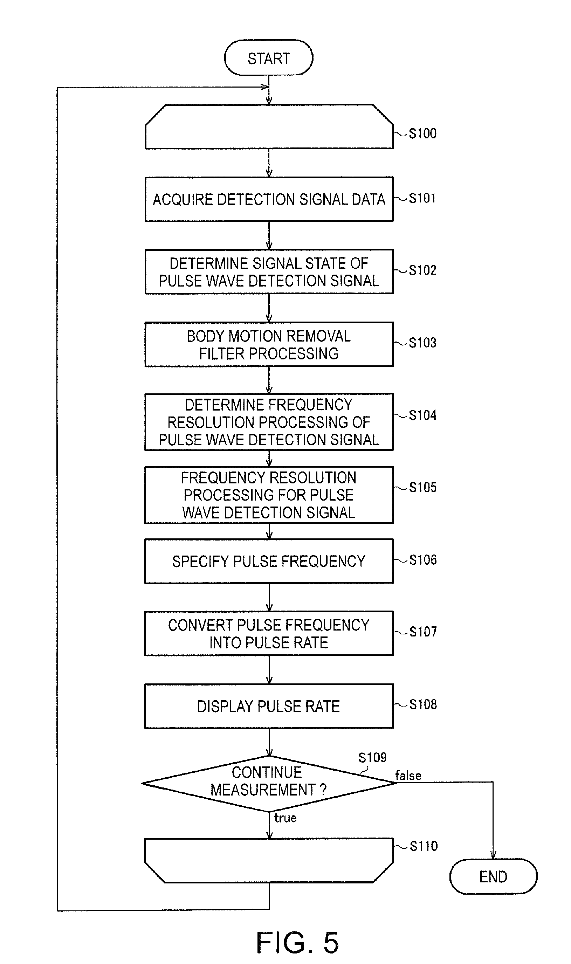

A f low of specif ic processing at the time when the pulsation detecting device 100 measures a pulse rate as pulsation information is shown in a flowchart of Fig. 5. The detecting unit 200 connected to the pulsation detecting device 100 includes an acceleration sensor as the body motion sensor 221 besides the pulsation sensor 211.

First, the pulsation detecting device 100 acquires a pulse wave detection signal and an acceleration detection signal serving as a body motion detection signal from the detecting unit 200 (S101). The pulsation detecting device 100 determines a signal state of the pulse wave detection signal on the basis of the pulse wave detection signal (S102). A specific determination method for the signal state is explained below.

Subsequently, the pulsation detecting device 100 applies body motion noise removal filter processing to the pulse wave detection signal using the acceleration detection signal (S103). The pulsation detecting device 100 determines, on the basis of the determined signal state, a frequency resolution processing applied to the pulse wave detection signal (S104). The pulsation detecting device 100 actually applies the frequency resolution processing to the pulse wave detection signal (S105) and specifies a pulse frequency on the basis of a result of the frequency resolution processing (S106). The pulsation detecting device 100 converts the pulse frequency into a pulse rate (S107) and displays the pulse rate on the displayunit 70 (S108). Finally, thepulsationdetectingdevice 100 determines whether measurement is continued (S109). When it is determined that the measurement is not continued, the pulsation detecting device 100 ends the processing. On the other hand, when it is determined that the measurement is continued, the pulsation detecting device 100 repeats the processing in steps S100 to S110.

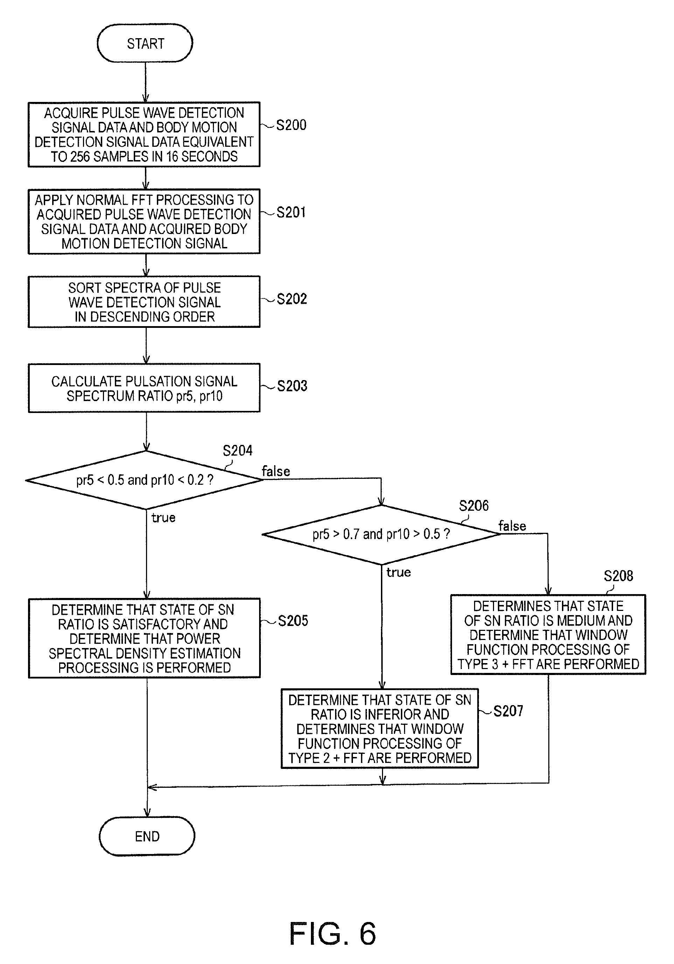

A specific example of the determination method for the signal state in step S102 is explained with reference to a flowchart of Fig. 6.

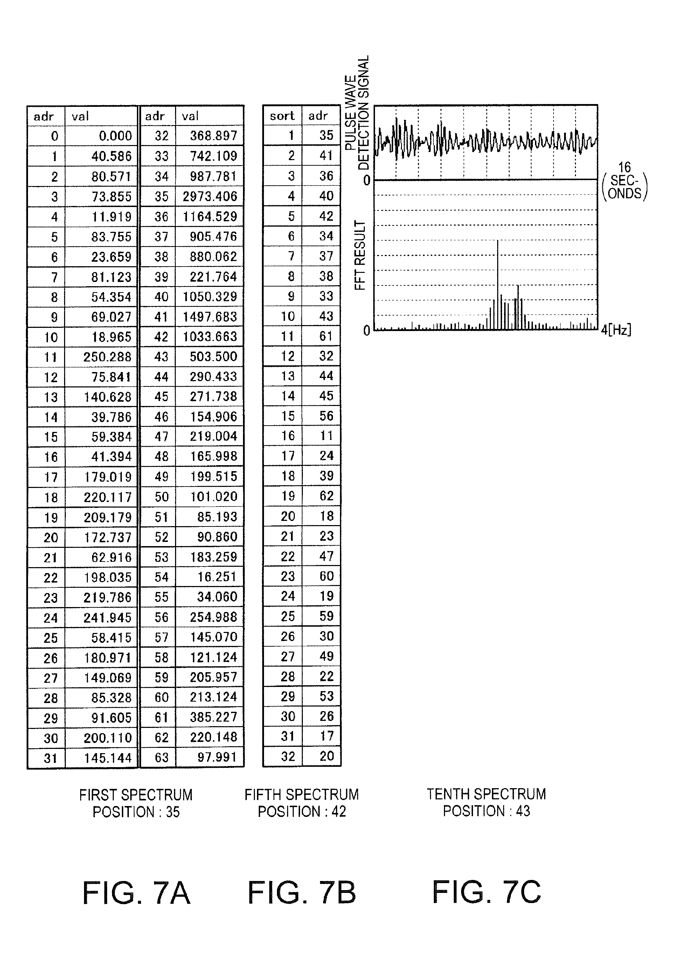

First, the pulsation detecting device 100 acquires data of the pulse wave detection signal and the body motion detection signal (the acceleration detection signal) equivalent to 256 samples in 16 seconds (S200). The pulsation detecting device 100 applies normal FFT processing to the acquired pulse wave detection signal and the acquired body motion detection signal (acceleration detection signal) (S201) and creates a table shown in Fig. 7(A) for each spectrum. In Fig. 7(A), adr indicates a spectrum number and val indicates a spectrum value. Further, as shown in Fig. 7 (B), the pulsation detecting device 100 sorts spectra of the pulse wave detection signal in a descending order (S202).

The pulsation detecting device 100 calculates a pulsation signal spectrum ratio (S203). Specifically, the pulsation detecting device 100 calculates a ratio of the magnitude of a maximum spectrum and the magnitude of a predetermined spectrum (e.g. , a fifth or tenth largest; not limited to fifth or tenth) among the other spectra. When the ratio is small, the pulsation detecting device 100 determines that unsteady noise is little and the pulse wave detection signal is formed by limited frequency components. That is, the pulsation detecting device 100 determines that an SN ratio is high (a satisfactory state) because the pulse wave detection signal does not include unnecessary frequency components and noise is little. On the other hand, when the ratio is large, the pulsation detecting device 100 determines that unsteady noise is large or frequency fluctuation is larger in an observation period. That is, the pulsation detecting device 100 determines that the SN ratio is low (an inferior state).

Specifically, the pulsation detecting device 100 determines whether a ratio pr5 of the maximum spectrum and the fifth largest spectrum is smaller than 0.5, which is a first threshold, and a ratio pr10 of the maximum spectrum and the tenth largest spectrum is smaller than 0.2, which is a second threshold (S204).

When determining that the ratio pr5 of the maximum spectrum and the fifth largest spectrum is smaller than 0.5, which is the first threshold, and the ratio pr10 of the maximum spectrum and the tenth largest spectrum is smaller than 0.2, which is the second threshold, the pulsation detecting device 100 determines (estimates) that the state of the SN ratio is satisfactory (the SN ratio is high) and determines that the power spectral density estimation processing is performed as the frequency resolution processing (S205).

For example, in an example shown in Fig. 7(A) to Fig. 7(C), pr5 is calculated as pr5=1033.663/2973.406=0.3476360 and pr10 is calculated as pr10=503.500/2973.406=0.1693344. Since pr5 is smaller than 0.5 and pr10 is smaller than 0.2, it is determined that the state of the SN ratio is satisfactory. Note that a time waveform of a pulse wave detection signal at this point and an FFT result are as shown in Fig. 7(C).

On the other hand, when the determination condition is not satisfied in step S204, the pulsation detecting device 100 determines whether the ratio rp5 of the maximum spectrum and the fifth largest spectrum is smaller than 0.7, which is a third threshold, and the ratio pr10 of the maximum spectrum and the tenth largest spectrum is smaller than 0.5, which is a fourth threshold (S206).

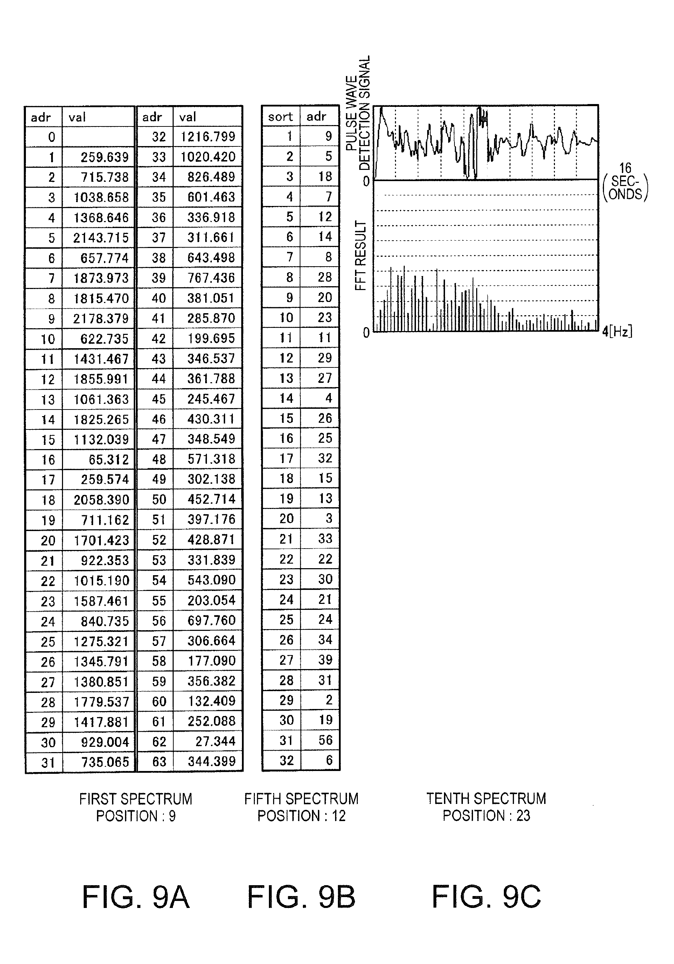

When determining that the ratio rp5 of the maximum spectrum and the fifth largest spectrum is larger than 0.7, which is the third threshold, and the ratio pr10 of the maximum spectrum and the tenth largest spectrum is larger than 0.5, which is the fourth threshold, the pulsation detecting device 100 determines (estimates) that the state of the SN ratio is inferior (the SN ratio is low) and determines that the window function processing by the asynchronous window function of Type 2 and the FFT are performed as the frequency resolution processing (S207). Specifically, an example shown in Fig. 9(A) to Fig. 9(C) corresponds to this case.

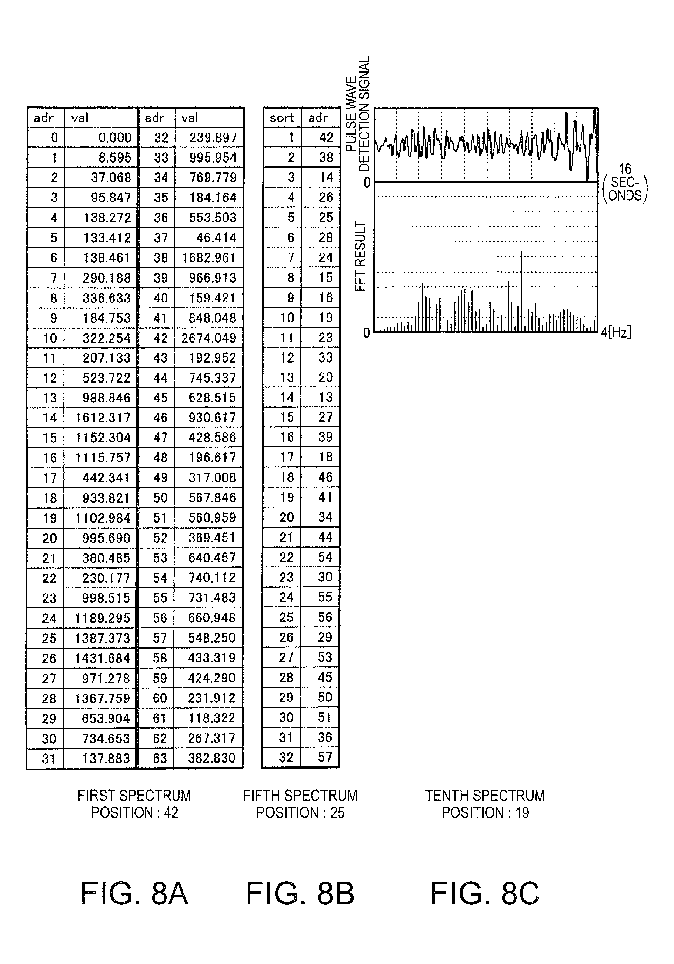

On the other hand, when the determination condition is not satisfied in step S206, the pulsation detecting device 100 determines (estimates) that the state of the SN ratio is medium and determines that the window function processing by the asynchronous window function of Type 3 and the FFT are performed as the frequency resolution processing (S208). Specifically, an example shown in Fig. 8 (A) to Fig. 8(C) corresponds to this case.

The determination method for the signal state is not limited to the method explained above. The determination of the signal state may be performed by any method.

The signal analyzing device 100 in this embodiment explained above includes the signal acquiring unit 110 that acquires a detection signal from the detecting unit 200 including the sensor and the signal processing unit 130 that performs the window function processing for sampling data $D_0$ to $D_N$ (N is a positive integer equal to or larger than 2) and the Fourier transform processing for data after the window function processing as the frequency resolution processing. Among the sampling data $D_0$ to $D_N$, jth sampling data $D_j$ is data sampled at timing later than timing when ith sampling data $D_i$ is sampled (integers i and j are integers satisfying $0 \leq i < N/2 < j \leq N$). The signal processing unit 130 performs the window function processing using the asymmetrical window function, a window function value h(j) of which corresponding to the jth sampling data $D_j$ is a maximum and a window function value h(i) of which corresponding to the ith sampling data $D_i$ is a value smaller than the window function value h(j). Note that the window function processing is performed by the window-function processing unit 133 in the signal processing unit 130 and the Fourier transform processing is performed by the Fourier-transform processing unit 135 in the signal processing unit 130.

The frequency resolution processing is processing for resolving (analyzing) a frequency included in a time signal. There are various methods for the frequency resolution processing. In this embodiment, the window function processing and the Fourier transform processing are performed as the frequency resolution processing. As explained above, the power spectral density estimation processing and the frequency resolution processing may be performed instead of the window function processing and the Fourier transform processing.

The sampling data $D_0$ to $D_N$ of the detection signal are (N+1) samples sampled from the detection signal. N only has to be a positive integer equal to or larger than 2. When the FFT is performed as explained above, the number of samples needs to be the power of 2. In this case, for example, N+1=2, 4, 8, 16, 32, 64, 128, 192, 256, 512, 1024, 2048, 4096, and the like. However, when Fourier transform other than the FFT is performed, the number of samples does not have to be the power of 2.

In this embodiment, the asymmetrical window function, the window function value h(j) of which corresponding to the jth sampling data $D_j$ is the maximum and the window function value h(i) of which corresponding to the ith sampling data $D_i$ is the value smaller than the window function value h(j), is used. The integers i and j are integers satisfying 0≤i<N/2<j≤N.

That is, in this embodiment, a signal value of a sample with 0≤i<N/2 is further compressed than a signal value of a sample with N/2<j≤N by setting the window function value h(i) corresponding to the ith sampling data $D_i$ smaller than the window function value h(j) corresponding to the jth sampling data $D_j$.

For example, in the example shown in Fig. 3, N+1=256. The integer i is an integer satisfying 0≤i≤127 and the integer j is an integer satisfying 127≤j≤255. Therefore, the window function value h (j) is maximized on the right side of the graph of Fig. 3 and the window function value h(i) on the left side of the graph is always smaller than h(j). All the window functions of Type 1 to Type 3 as explained above satisfy this condition.

Consequently, in the window function processing, it is possible to compress a sample in the past compared with a recent sample. Therefore, compared with when the window function processing is not performed or when the window function processing is performed using the symmetrical window function, it is possible to improve the real time property of a frequency resolution processing result (a pulse rate). When the window function processing is not performed and when the real time property equivalent to the real time property obtained when the window function processing is performed using the symmetrical window function is sufficient in specifications, it is possible to further extend the sampling period. Therefore, it is possible to obtain a frequency distribution with higher frequency resolution.

As explained above, if the values at both the ends of the sampling data are 0, the sampling data is closer to the signal waveform that can be represented by the periodic function. Therefor, it is possible to improve the side lobe characteristic when the Fourier transform is performed.

Therefore, the signal processing unit 130 may perform the window function processing using the asynchronous window function, the window function value h(N) corresponding to the Nth sampling data $D_N$ among the sampling data $D_0$ to $D_N$ of which is the minimum.

That is, in the example shown in Fig. 3, the window function value h(255) for a 255th sample number is set to 0. In this case, a signal value multiplied with the window function value h(255) is 0. The window functions of Type 1 and Type 2 shown in Fig. 3 have such a characteristic.

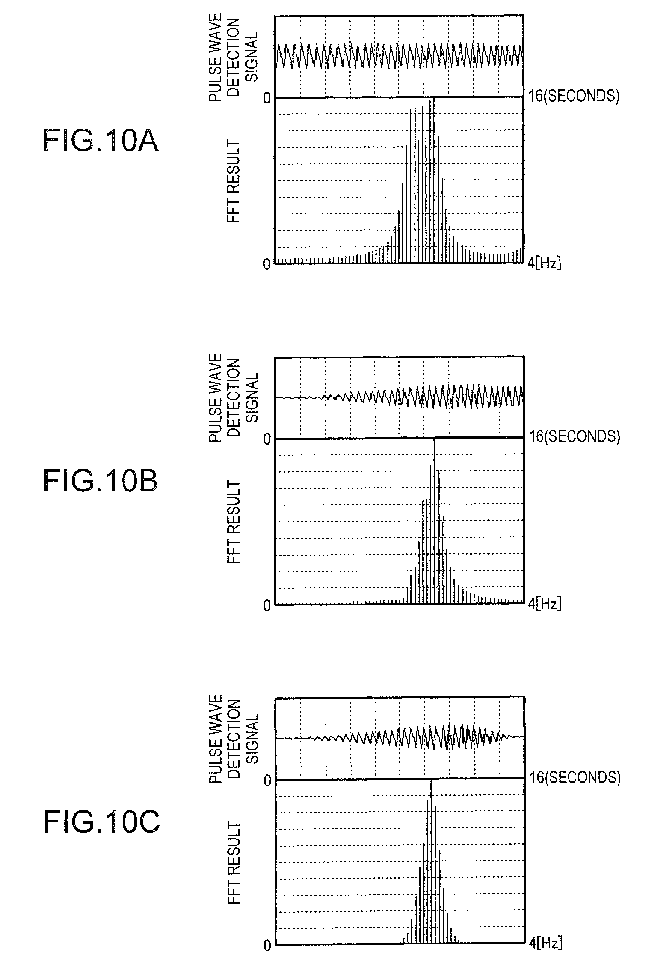

Consequently, compared with when the window function processing is not performed, it is possible to, for example, improve the side lobe characteristic. Compared with when the window function processing is performed using the symmetrical window function, it is possible to, for example, improve the real time property of a frequency resolution processing result (a pulse rate). A specific application result is explained below with reference to Fig. 10(A) to Fig. 10(C).

A specific expression of the asynchronous window function of Type 1 is illustrated.

The signal processing unit 130 may apply the window function processing to pth sampling data Dp among the sampling data$D_0$ to $D_N$ (integers p, M, andNare integers satisfying 0≤p≤3M-1 and N=4M-1) according to Expression (1) shown below and apply the window function processing to qth sampling data Dq (integers q, M, andNare integer satisfying 3M≤q≤4M-1 and N=4M-1) according to Expression (2) shown below.

[Math. 1]

$$h(p) = \sin\left(\frac{\pi p}{2(3M-1)}\right) \quad \cdots \quad (1)$$

[Math. 2]

$$h(q) = \cos\left(\frac{\pi(q-3M+1)}{2M}\right) \quad \cdots \quad (2)$$

That is, in the example shown in Fig. 3, since N+1=256 and M=64, the signal processing unit 130 applies the window function processing to the sampling data $D_p$ with 0≤p≤191 according to Expression (1) and applies the window function processing to the sampling data Dq with 192≤q≤255 according to expression (2).

The asynchronous window function of Type 1 has a small compression degree of a signal compared with the window function of Type 2. Therefore, even when a level of the pulse wave detection signal is low, a power spectrum value indicating pulsation tends to be clear. It is possible to, for example, easily specify a pulse frequency.

A specific expression of the asynchronous window function of Type 2 is illustrated.

The signal processing unit 130 may apply the window function processing to the pth sampling data Dp among the sampling data $D_0$ to $D_N$ (integers p, M, andNare integers satisfying 0≤p≤3M-1 and N=4M-1) according to Expression (3) shown below and apply the window function processing to the qth sampling data Dq (integersq, M, andNare integer satisfying 3M≤q≤4M-1 and N=4M-1) according to Expression (4) shown below.

[Math. 3]

$$h(p) = \frac{1}{2}\left\{1 - \cos\left(\frac{\pi p}{3M - 1}\right)\right\} \quad \cdots \quad (3)$$

[Math. 4]

$$h(q) = \frac{1}{2}\left\{1 + \cos\left(\frac{3\pi(q - 3M + 1)}{3M - 1}\right)\right\} \quad \cdots \quad (4)$$

That is, in the example shown in Fig. 3, since N+1=256 and M=64, the signal processing unit 130 applies the window function processing to the sampling data $D_p$ with 0≤p≤191 according to Expression (3) and applies the window function processing to the sampling data Dq with 192≤q≤255 according to expression (4).

Consequently, in the Nth sampling data, it is possible to, for example, set not only a signal value but also a gradient to 0.

In Fig. 10(A) to Fig. 10(C), results of the kinds of frequency resolution processing are shown. Upper figures in the figures show time waveforms of pulse wave detection signals in 16 seconds. Lower figures of the figures show FFT results of the pulse wave detection signals shown in the upper figures. Fig. 10(A) shows an example in which the window function processing is not performed. Fig. 10(B) shows an example in which the window function processing is performed using the asynchronous window function of Type 3 explained below. Fig. 10 (C) shows an example in which the window function processing is performed using the asymmetrical window function of Type 2.

As indicated by the FFT results shown in Fig. 10(A) to Fig. 10(C), when the FFT is applied to a signal after the window function processing by the asynchronous window function, a distribution of power spectra is narrowed. That is, the side lobe is suppressed and the side lobe characteristic is improved. This is an effect by compression a signal portion in the past. As a result, it is easier to specify a frequency indicating a pulse. Further, when the results are compared, it is seen that the result obtained when the asymmetrical window function of Type 2 is used (Fig. 10(C)) has the best side lobe characteristic among the three FFT result.

Therefore, if the asynchronous window function of Type 2 is used, it is possible to further improve the side lobe characteristic. It is possible to, for example, specify a pulse frequency even in a state in which noise is serious.

As explained above, for example, when it is determined that the state of the SN ratio is satisfactory, that is, when it is determined that body motion noise and mixing of sudden disturbance are little, since the side lobe does not often occurs, it is unnecessary to always improve the side lobe characteristic. In this case, it is desirable to perform the window function processing with more importance placed on the real time property rather than improving the side lobe characteristic.

Therefore, the signal processing unit 130 may perform the window function processing using the asynchronous window function, the window function value h (N) of which corresponding to the Nth sampling data $D_N$ among the sampling data $D_0$ to $D_N$ is the maximum.

For example, if the window function value h(N)=1, the compression of the signal value of the Nth sampling data is not performed. Even if h(N) is not 1, it is possible to set a compression ratio lowest with respect to the other sampling data.

Consequently, compared with not only when the window function processing is not performed and the Fourier transform is performed as shown in Fig. 10(A) but also when the window function processing is performed using the general window function or the asymmetrical window function of Type 1 or Type 2, it is possible to, for example, the real time property of the frequency resolution processing result (the pulse rate).

If the window function value is increased and the compression ratio is reduced for not only the Nth sampling data but also sampling data tracing back from the Nth sampling data by a predetermined number, the real time property of the frequency resolution processing result (the pulse rate) is improved.

Therefore, the signal processing unit 130 may perform the window function processing using the asynchronous window function , all the window function values h(k) to h(N) of which corresponding to the kth sampling data $D_k$ to the Nth sampling data $D_N$ (an integer k is an integer satisfying N/2<k<N) among the sampling data $D_0$ to $D_N$ are the maximum.

For example, in the example of the asymmetrical window function of Type 3 shown in Fig. 3, all window functions h(192) to h(255) corresponding to 192th to 255th sampling data are set to 1, which is the maximum.

Consequently, not only for the Nth sampling data but also for the sampling data tracing back from the Nth sampling data by the predetermined number, it is possible to, for example, maximize the window function value and reduce the compression ratio. Therefore, compared with when the window function processing is not performed, when the window function processing is performed using the symmetrical window function, or when the window function processing is performed using the asymmetrical window function of Type 1 or Type 2, it is possible to, for example, improve the real time property of the frequency resolution processing result (the pulse rate).

A specific expression of the asymmetrical window function of Type 3 is illustrated.

The signal processing unit 130 may apply the window functionprocessing to the pth sampling data $D_p$ among the sampling data $D_0$ to $D_N$ (integers p, M, and N are integers satisfying $0 \leq p \leq 3M-1$ and $N=4M-1$) according to Expression (5) shown below and apply the window function processing to the qth sampling data Dq (integersq, M, andNare integer satisfying $3M \leq q \leq 4M-1$ and $N=4M-1$) according to an expression h(q)=1.

[Math. 5]

$$h(p) = \frac{1}{2}\left\{1 - \cos\left(\frac{\pi p}{3M-1}\right)\right\} \quad \cdots \quad (5)$$

That is, in the example shown in Fig. 3, since N+1=256 and M=64, the signal processing unit 130 applies the window function processing to the sampling data Dp with $0 \leq p \leq 191$ according to Expression (5) and applies the window function processing to the sampling data Dq with $192 \leq q \leq 255$ according to the expression h(q)=1.

Consequently, it is possible to, for example, maximize the window function value not only for the Nth sampling data but also for sampling data tracing back from the Nth sampling data by a predetermined number. For example, it is seen that, in a graph shown in the upper figure of Fig. 10(B), a signal value of 64 samples on the right side of the graph is not compressed from an original waveform shown in the upper figure of Fig. 10(A).

Note that the window function value may be calculated in advance and retained in an array variable rather than being calculated every time the window function processing is performed. Consequently, it is possible to reduce computational complexity.

Incidentally, the asynchronous window functions of Type 1 to Type 3 are desirably properly used according to signal states as explained above. The frequency resolution processing is not limited to the processing for performing the window function processing and the FFT. The power spectral density estimation processing is sometimes desirably performed.

Therefore, the pulsation detecting device 100 in this embodiment may include the signal processing unit 130 that performs the frequency resolution processing on the basis of the pulse wave detection signal from the pulse-wave detecting unit 210 and the body motion detection signal from the body-motion detecting unit 220, the pulsation-information calculating unit 170 that calculates pulsation information on the basis of a result of the frequency resolution processing, and the signal-state determining unit 150 that applies the determination processing for the signal state to at least one of the pulse wave detection signal and the body motion detection signal. The signal processing unit 130 performs a different kind of the frequency resolution processing according to a determination result of a signal state by the signal-state determining unit 150.

Consequently, it is possible to, for example, a different kind of the frequency resolutionprocessing according to a signal state.

In the window function processing, the window function in use should be switched according to whether a lot of noise is included in the pulse wave detection signal. Therefore, a signal state may be determined on the basis of determination whether an SN ratio can be estimated high or can be estimated low.

That is, the signal processing unit 130 may perform first frequency resolution processing when it is determined that the pulse wave detection signal is ina first signal state and perform second frequency resolution processing when it is determined that the pulse wave detection signal is in a second signal state in which the SN ratio is higher than the SN ratio in the first signal state.

Consequently, it is possible to, for example, perform a different kind of the frequency resolution processing according to a signal state in which the SN ratio is different.

Depending on a signal state, it is also possible to perform the power spectral density estimation processing and the like as the frequency resolution processing without performing the window function processing as the example explained above.

That is, the signal analyzing device 100 in this embodiment may include the signal-state determining unit 150 that performs the determination processing for the signal state concerning a detection signal acquired by the signal acquiring unit 110. The signal processing unit 130 may perform the window function processing by the asymmetrical window function and the Fourier transform processing for data after the window function processing as the frequency resolution processing when it is determined that the signal state is the first signal state and perform the power spectral density estimation processing for the detection signal as the frequency resolution processing when it is determined that the signal state is the second signal state.

In other words, when the signal analyzing device 100 is limited to the pulsation detecting device 100, the signal processing unit 130 may perform the window function processing for sampling data of the pulse wave detection signal and the Fourier transform processing for data after the window function processing as the first frequency resolution processing when it is determined that the pulse wave detection signal is in the first signal state and perform the power spectral density estimation processing as the second frequency resolution processing when it is determined that the pulse wave detection signal is in the second signal state.

The power spectral density estimation processing is a calculation algorithm that can obtain, compared with calculating a frequency distribution with the normal FFT, frequency resolution and a distinction property of the frequency equivalent to those obtained when the FFT processing is applied to a long-term observation signal even if only a shorter-term observation signal is obtained. In fields other than the pulse detection, the power spectral density estimation processing is often used in, for example, an earthquake occurrence cycle analysis and an ocean tide analysis.

Specifically, when the power spectral density estimation processing is performed, for example, it is possible to, for example, calculate a pulse rate using only a signal waveform about 2 seconds before the present. In that case, it is possible to neglect a frequency component included in a pulse wave detection signal further in the past than 2 seconds.

Consequently, it is possible to perform the frequency analysis processing with a high side lobe suppression effect in the first signal state in which the SN ratio is higher than the SN ratio in the second signal state and perform the frequency resolution processing with more importance placed on the real time property in the second signal state in which the SN ratio is higher than the SN ratio in the first signal state.

Further, when it is determined that the pulse wave detection signal is in the first signal state, the signal processing unit 130 may perform the window function processing using the asynchronous window function, the window function value $h(j)$ of which corresponding to the jth sampling data $D_j$ is a maximum and the window function value $h(i)$ of which corresponding to the ith sampling data $D_i$ is a value smaller than the window function value $h(j)$.

Consequently, compared with when the window function processing is not performed or when the window function processing is performed using the symmetrical window function, it is possible to, for example, improve the real time property of a frequency resolution processing result (a pulse rate).

Further, the signal processing unit 130 may perform the window function processing by the first asymmetrical window function when it is determined that the signal state is the first signal state and perform the window function processing by the second asymmetrical window function different from the first asymmetrical window function when it is determined that the signal state is the second signal state.

In other words, when the signal analyzing device 100 is limited to the pulsation detecting device 100, the signal processing unit 130 may perform the window function processing for the sampling data of the pulse wave detection signal, in which a first window function is used, and the Fourier transform processing for first data after the window function processing as the first frequency resolution processing when it is determined that the pulse wave detection signal is in the first signal state and perform the window function processing for the sampling data of the pulse wave detection signal, in which a second window function different from the first window function is used, and the Fourier transform processing for second data after the window function processing as the second frequency resolution processing when it is determined that the pulse wave detection signal is in the second signal state.

Further, when it is determined that the pulse wave detection signal is in the first signal state, the signal processing unit 130 may perform the window function processing using the asymmetrical window function, the window function value $h(j)$ of which corresponding to the jth sampling data $D_j$ is a maximum and the window function value $h(i)$ of which corresponding to the ith sampling data $D_i$ is a value smaller than the window function value $h(j)$, as the first window function when it is determined that the pulse wave detection signal is in the first signal state and perform the window function processing using the second window function, which is the asymmetrical window function different from the first window function, when it is determined that the pulse wave detection signal is in the second signal state.

That is, the signal processing unit 130 may perform the window function processing respectively using the different asymmetrical window functions when the signal state is the first signal state and when the signal state is the second signal state.

For example, it is possible to, for example, perform the window function processing using the asymmetrical window function of Type 3 as the first window function when the signal state is the first signal state and perform the window function processing using the asymmetrical window function of Type 2 as the second window function when the signal state is the second signal state. Note that the symmetrical window function may be used depending on a case.

Consequently, it is possible to, for example, perform a different kind of the window function processing according to the signal state. Further, it is possible to, for example, change the asymmetrical window function used in the window function processing according to the signal state.

When determining a signal state on the basis of a state of the SN ratio as explained above, it is necessary to calculate the SN ratio. However, since it is difficult to directly calculate the SN ratio, it is necessary to estimate the SN ratio from other information.

Therefore, the signal-state determining unit 150 may calculate a ratio of spectrum values of a first spectrum indicating a maximum spectrum value and at least one second spectrum other than the first spectrum among frequency spectra obtained by the frequency resolution processing of the pulse wave detection signal and perform the determination processing for the signal state. Note that the spectrum-value calculating unit 151 of the signal-state determining unit 150 performs calculation processing for a spectrum value.

Specifically, the determinationprocessing for the signal state is as explained with reference to the flowchart of Fig. 6.

Consequently, it is possible to, for example, estimate the SN ratio and calculate a signal state.

5. Second Embodiment

5.1. Method

A method in a second embodiment is explained. As explained above, to improve the resolution of frequency resolution processing, it is necessary to extend a detection period (a sampling period) of a pulse wave detection signal used for performing pulse rate calculation processing once. When a pulse does not change during the detection period, since a specific spectrum clearly shows a peak value, it is relatively easy to specify a pulse frequency.

On the other hand, when a pulse rate greatly changes in the detection period, since spectra diffuse in a wide band, it is difficult to specify a peak spectrum. For example, in the detection period, when a subj ect starts jogging in a sitting state (a pulse is 60) and the pulse rises to about 150, pulse spectra diffuse in a section of 1 to 2.5 Hz. Since the magnitudes of respective frequency spectra are the same, it is more difficult to determine which frequency is a pulse frequency.

Therefore, the signal analyzing device (the pulsation detecting device) 100 in the second embodiment determines an exercise state on the basis of a body motion detection signal and selects a procedure of the frequency resolution processing according to the exercise state.

When it is determined that the exercise state is a rest state, that is, only a very small motion is detected in a body motion detection signal such as an acceleration detection signal, signal analyzing device (the pulsation detecting device) 100 performs the power spectral density estimation processing as the frequency resolution processing.

When it is determined that the exercise state is a steady exercise state, signal analyzing device (the pulsation detecting device) 100 performs the window function processing using the window function of Type 3 as the frequency resolution processing and applies the FFT to data after the window function processing. Specifically, the steady exercise state indicates a walking state, a jogging state, or the like and means a state in which the subject is moving the body at a substantially fixed cycle (rhythm). That is, the steady exercise state indicates a state in which the subject is performing an exercise having periodicity.

When it is determined that the exercise state is an unsteady exercise state, signal analyzing device (the pulsation detecting device) 100 performs the window function processing using the window function of Type 2 or Type 3 as the frequency resolution processing and applies the FFT to data after the window function processing. Specifically, the unsteady exercise state indicates an exercising state, a ball game playing state, or the like and means a state in which the subject moves the body at an unfixed cycle (an unspecific cycle). That is, the unsteady exercise state indicates a state in which the subject is performing an exercise not having periodicity.

When it is detected that the exercise state changes from the rest state to the steady exercise state or the unsteady exercise state, that is, when it is detected that the subject starts an exercise, signal analyzing device (the pulsation detecting device) 100 performs the window function processing using the window function of Type 1 or Type 2 as the frequency resolution processing and applies the FFT to data after the window function processing.

On the other hand, when it is detected that the exercise state changes from the steady exercise state or the unsteady exercise state to the rest state, that is, when it is detected that the subject stops the exercise, signal analyzing device (the pulsation detecting device) 100 performs the power spectral density estimation as the frequency resolution processing.

5.2. Flow of processing

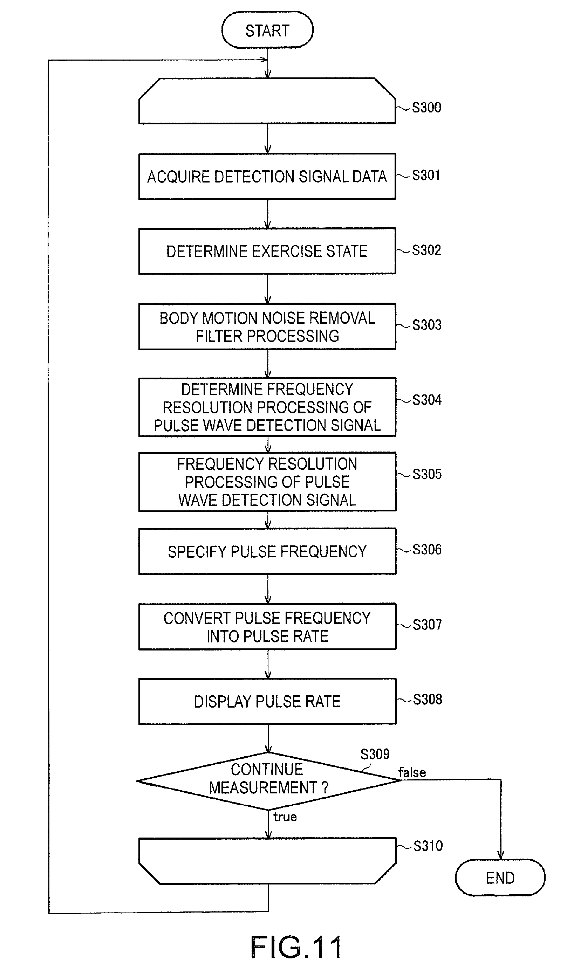

A flow of specific processing performed when the pulsation detecting device 100 measures a pulse rate as pulsation information is shown in a flowchart of Fig. 11. The detecting unit 200 connected to the pulsation detecting device 100 includes an acceleration sensor as the body motion sensor 221 besides the pulse wave sensor 211.

A flow of basic processing is the same as the flow of the processing shown in Fig. 5 in the first embodiment.

However, step S302 corresponding to step S102 is different. In this embodiment, the pulsation detecting device 100 determines an exercise state of the subject on the basis of an acceleration detection signal (S302).

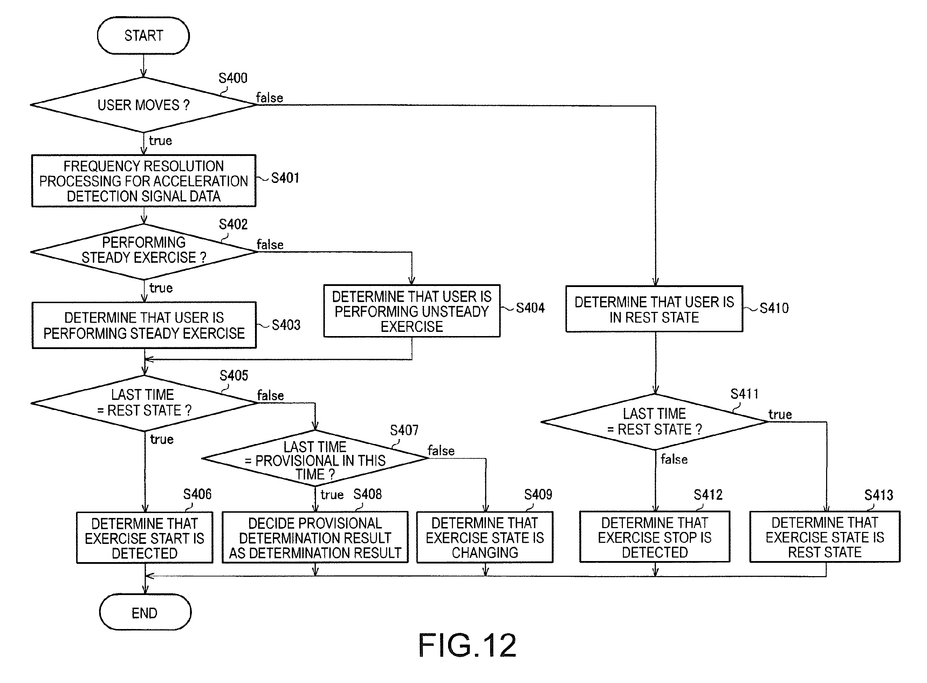

A specific example of a determination method for an exercise state in step S302 is explained with reference to a flowchart of Fig. 12.

First, the pulsation detecting device 100 determines, on the basis of the acceleration detection signal, whether a user moves (S400).

When determining that the user moves, the pulsation detecting device 100 performs the frequency resolution processing for acceleration detection signal data (S401). Note that the frequency resolution processing performed here is not frequency resolution processing applied to a pulse wave detection signal. The pulsation detecting device 100 determines whether the user is performing the steady exercise (S402). When determining that the exercise state is the steady exercise state, the pulsation detecting device 100 determines the steady exercise state as a provisional determination result of the exercise state (S403).

On the other hand, when determining that the exercise state is not the steady exercise state, the pulsation detecting device 100 determines the unsteady exercise state as a provisional determination result of the exercise state (S404).

After step S403 or S404, the pulsation detecting device 100 determines whether the exercise state during the last determination is the rest state (S405).

When the exercise state during the last determination is the rest state, the pulsation detecting device 100 determines that an exercise start is detected (S406) and ends the exercise state determination processing.

On the other hand, when determining that the exercise state during the last determination is not the rest state, the pulsation detecting device 100 determines whether the exercise state during the last determination coincides with the provisional exercise state determined in step S403 (S407). That is, the pulsation detecting device 100 determines whether the exercise changes.

When the exercise state during the last determination coincides with the provisional exercise state, since it is considered that the exercise state does not change and the user is continuing the same exercise, the pulsation detecting device 100 decides the provisional exercise state as a determination result (S408) and ends the exercise state determination processing.

On the other hand, when the exercise state during the last determination does not coincide with the provisional exercise state, since it is considered that the user is not continuing the same exercise, the pulsation detecting device 100 determines that the exercise state is changing (S409) and ends the exercise state determination processing.

Further, when determining in step S400 that the user does not move, the pulsation detecting device 100 determines the provisional determination result as the rest state (S410). When the exercise state during the last determination is not the rest state, the pulsation detecting device 100 determines that an exercise stop is detected (S412) and ends the exercise state determination processing.

On the other hand, when the exercise state during the last determination is the rest state, the pulsation detecting device 100 determines that the user does not move, determines that the exercise state is the rest state (S413), and ends the exercise state determination processing. The flow of the determination processing for the exercise state is as explained above.

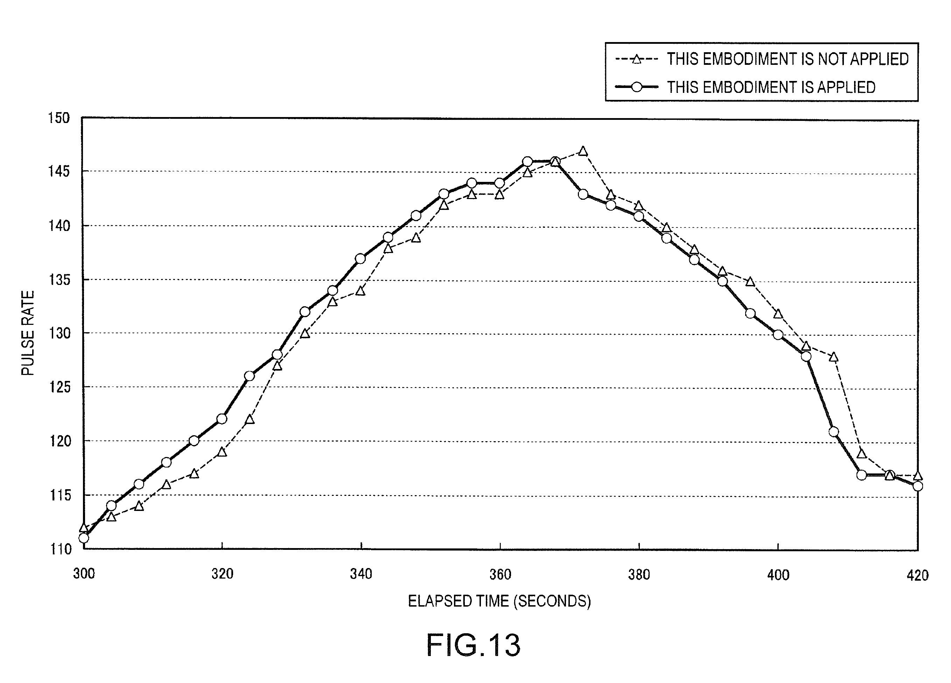

A graph of comparison of measurement results of pulse rates obtained when this embodiment is applied and when this embodiment is not used is shown in Fig. 13.

The measurement results shown in Fig. 13 indicate temporal transitions of pulse rates at the time when the user jogs about 1 minute from a walking state (around 300 seconds) and walks again from around 360 seconds. By applying this embodiment, a rise and a fall in the pulse rates are considered to be observed at earlier timing with respect to the elapse of time.

In this embodiment explained above, the detecting unit 200 may further include a second sensor besides a first sensor functioning as a sensor. In this case, the signal acquiring unit 110 may acquire, from the detecting unit 200, a first detection signal based on a first sensor signal acquired by the first sensor and a second detection signal based on a second sensor signal acquired by the second sensor.

The signal-state determining unit 150 may perform, as determination processing, processing for determining a signal state of the second detection signal. Further, the signal processing unit 130 may apply the window function processing to the first detection signal using a first asynchronous window function as the asynchronous window function when it is determined that the signal state of the second detection signal is a first signal state and apply the window function processing to the first detection signal using a second asynchronous window function different from the first asynchronous window function when it is determined that the signal state of the second detection signal is a second signal state.

Consequently, it is possible to, for example, change, according to the signal state of the second detection signal different from the first detection signal, the asynchronous window function used in the window function processing and perform the frequency resolution processing.

The signal processing unit 130 may apply the window function processing for the first detection signal using a first asynchronous window function, in which the first asynchronous window function is used as the asynchronous window function, and the Fourier transform processing for data after the window function processing as the frequency resolution processing when it is determined that the signal state of the second detection signal is the first signal state and perform the power spectral density estimation processing for the first detection signal as the frequency resolution processing when it is determined that the signal state of the second detection signal is the second signal state.

Consequently, it is possible to, for example, determine a signal state according to the signal state of the second detection signal and, for example, when the signal state is the first signal state in which an SN ratio is lower than an SN ratio in the second signal state, perform the frequency resolution processing with high side lobe suppression effect and, when the signal state is the second signal state in which the SN ratio is higher than the SN ratio in the first signal state, perform the frequency resolution processing with more importance placed on the real time property.

The signal-state determining unit 150 may perform, as the determination processing, processing for determining the exercise state of the subj ect on the basis of the second detection signal. The signal processing unit 130 may determine, on the basis of a determination result of the exercise state, processing applied to the first detection signal. Note that the exercise-state determining unit 153 included in the signal-state determining unit 150 performs the determination processing for the exercise state.

In other words, when the signal analyzing device 100 is limited to the pulsation detecting device 100, the signal-state determining unit 150 may perform, as the determination processing for the signal state, processing for determining an exercise state of the subject on the basis of a body motion detection signal. The signal processing unit 130 may perform a different kind of the frequency resolution processing according to the exercise state.

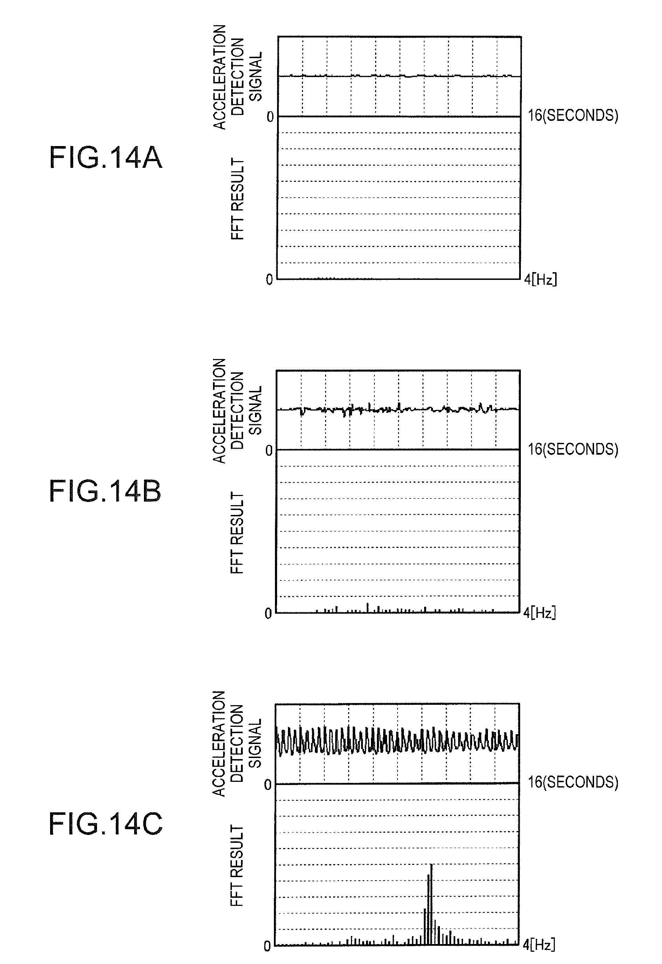

As explained above, specifically, as the exercise state, there are the rest state, the steady exercise state, the unsteady exercise state, and the like. An example of specific determination processing for an exercise state, in which the acceleration sensor is used as a body motion sensor, is explained with reference to Fig. 14 (A) to Fig. 14(C). Fig. 14 (A) to Fig. 14(C) show spectra of acceleration detection signals in the exercise states. Upper figures of the figures show time waveforms of acceleration detection signals in 16 seconds. Lower figures of the figures show FFT results of the acceleration detection signals shown in the upper figures.

First, in the case of Fig. 14(A), since the amplitude of the acceleration detection signal is equal to or smaller than a predetermined threshold, it is determined that the exercise state is the "rest state". In the case of Fig. 14 (B), since the amplitude of the acceleration detection signal is equal to or larger than the predetermined threshold, it is determined that "the user moves". In addition, since a relatively conspicuously large power spectrum is absent in a specific frequency region, it is determined that the exercise state is the "unsteady exercise state". Further, in the case of Fig. 14 (C), as in the case of Fig. 14 (B), since the amplitude of the acceleration detection signal is equal to or larger than the predetermined threshold, it is determined that "the user moves". In addition, since a relatively conspicuously large power spectrum is present in the specific frequency region, it is determined that the exercise state is the "steady exercise state".

Consequently, it is possible to, for example, perform a different kind of the frequency resolution processing according to the exercise state.

Specifically, the signal processing unit 130 may perform the first frequency resolution processing when it is determined that the exercise state is the steady exercise state and perform the second frequency resolution processing when it is determined that the exercise state is the unsteady exercise state.

Consequently, it is possible to, for example, perform different kinds of the frequency resolution processing when the pulse wave detection signal has periodicity and when the pulse wave detection signal does not have periodicity.

Further, the signal processing unit 130 may perform the window function processing for the sampling data of the pulse wave detection signal, in which the first window function is used, and the Fourier transform processing for first data after the window function processing as the first frequency resolution processing when it is determined that the exercise state is the steady exercise state and perform the window function processing for the sampling data of the pulse wave detection signal, in which the second window function different from the first window function is used, and the Fourier transform processing for second data after the window function processing as the second frequency resolution processing when it is determined that the exercise state is the unsteady exercise state.

Consequently, it is possible to, for example, perform the window function processing respectively using the different window functions when the pulse wave detection signal has periodicity and when the pulse wave detection signal does not have periodicity.

The jth sampling data $D_j$ among the sampling data $D_0$ to $D_N$ (N is a positive integer equal to or larger than 2) of the pulse wave detection signal is data sampled at timing later than timing when the ith sampling data $D_i$ is sampled (integers i and j are integers satisfying $0 \le i < N/2 < j \le N$).

In this case, the signal processing unit 130 may perform the window function processing using the asymmetrical window function, the window function value h (j) of which corresponding to the jth sampling data $D_j$ is a maximum and the window function value h(i) of which corresponding to the ith sampling data $D_i$ is a value smaller than the window function value h(j), as the first window function when it is determined that the exercise state is the steady exercise state and perform the window function processing using the second window function, which is the asymmetrical window function different from the first window function, when it is determined that the exercise state is the unsteady exercise state.

That is, the window function used when the exercise state is the steady exercise state and the window function used when the exercise state is the unsteady exercise state may be respectively different asynchronous window functions.

Consequently, it is possible to, for example, perform the window function processing respectively using the different asymmetrical window functions when the pulse wave detection signal has periodicity and when the pulse wave detection signal does not have periodicity.

When the exercise state is the rest state, noise due to a body motion or the like is hardly included in the pulse wave detection signal.

Therefore, the signal processing unit 130 may perform the first frequency resolution processing when it is determined that the exercise state is the rest state and perform the second frequency resolution processing when it is determined that the exercise state is the steady exercise state or the unsteady exercise state.

Consequently, it is possible to, for example, perform different kinds of the frequency resolution processing when the exercise state is the rest state and when the exercise state is the steady exercise state or the unsteady exercise state.

Specifically, the signal processing unit 130 may perform the power spectral density estimation processing as the first frequency resolution processing when it is determined that the exercise state is the rest state and perform the window function processing for the sampling data of the pulse wave detection signal and the Fourier transform processing for data after the window function processing as the second frequency resolution processing when it is determined that the exercise state is the in-exercise state.

Consequently, it is possible to, for example, perform the frequency resolution processing with a high suppression effect of a side lobe when the exercise state is the steady exercise state or the unsteady exercise state and perform the frequency resolution processing with more importance placed on the real time property when the exercise state is the rest state.

The signal processing unit 130 may perform the window function processing using the asymmetrical window function, the window function value h(j) of which corresponding to the jth sampling data $D_j$ is a maximum and the window function value h(i) of which corresponding to the ith sampling data $D_i$ is a value smaller than the window function value h(j), as the first window function when it is determined that the exercise state is the steady exercise state or the unsteady exercise state.

Consequently, it is possible to, for example, perform the window function processing by the asymmetrical window function when the exercise state is the steady exercise state or the unsteady exercise state and perform the frequency resolution processing with an improved real time property.

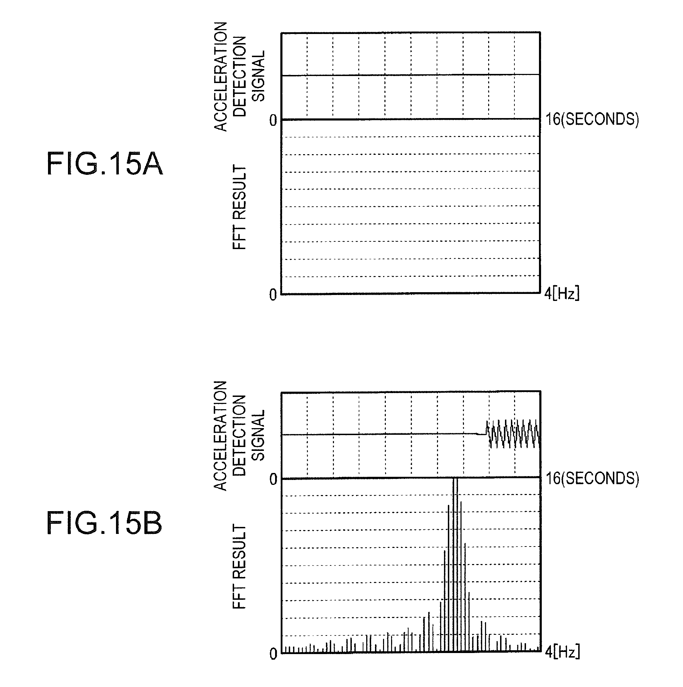

It is also possible to determined, on the basis of a spectrum of the acceleration detection signal, whether the user starts an exercise and switch, on the basis of a determination result, the frequency resolution processing to be executed. In Fig. 15 (A), a spectrum of the acceleration detection signal before an exercise start is shown. In Fig. 15(B), a spectrum of the acceleration detection signal immediately after the exercise start is shown. A specific determination method is as shown in the flowchart of Fig. 12. In this way, if a waveform of the acceleration detection signal is observed, it is possible to determine whether the user starts an exercise.

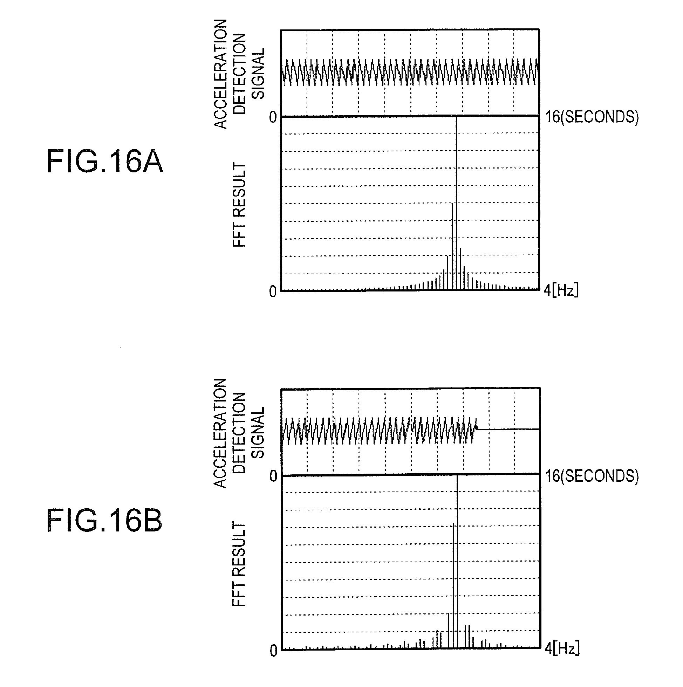

Further, it is also possible to determine, on the basis of a spectrum of the acceleration detection signal, whether user stops the exercise and switch, on the basis of a determination result, the frequency resolution processing to be executed. In Fig. 16(A), a spectrum of the acceleration detection signal during an exercise is shown. In Fig. 16(B), a spectrum of the acceleration detection signal immediately after the exercise stop is shown. A specific determination method is as shown in the flowchart of Fig. 12. In this way, if a waveform of the acceleration detection signal is observed, it is possible to determine whether the user stops the exercise.

A use of the second detection signal is not limited to the determination of a signal state.

That is, the signal processing unit 130 may apply signal processing to the first detection signal on the basis of the second detection signal.

Specifically, the signal processing unit 130 (the noise-reduction processing unit 131) may perform, as the signal processing described above, noise reduction processing for the first detection signal on the basis of the second detection signal.

In the pulsation detecting device 100, it is assumed that various kinds of noise are mixed in the pulse wave sensor signal acquired from the pulse wave sensor (or the pulse wave detection signal acquired from the pulse wave sensor). Therefore, if the noise reduction processing for reducing a body motion noise component or the like included in the pulse wave sensor signal is not performed, pulsation information to be detected is also affected by the noise. Therefore, it is likely that a value of the pulsation information does not accurately represent an actual pulsation of a wearer. In this case, if the pulsation information is simply output, the user is likely to make an error in judgment based on the pulsation information.

Therefore, the noise-reduction processing unit 131 may perform processing for reducing noise due to a body motion (a body motion noise component) from the pulse wave detection signal.

The body motion noise component (a body motion noise component signal) means a component signal indicating a capacity change of blood vessels that occurs because of a steady exercise, motion (body motion), or the like of a human among component signals included in the pulse wave sensor signal. For example, in the case of a pulse rate meter worn on an arm or a finger, because of the influence of an arm swing during walking or during jogging, a capacity change occurs in blood vessels according to rhythm of the swing of the arm. When the human performs a steady motion, the body motion noise component changes to a cyclic signal and changes to a component signal having the frequency of the motion. The body motion noise component has a characteristic that a correlation with a waveform of a signal output by the acceleration sensor worn around a wearing part of the pulse wave sensor is high.

In the pulse wave sensor signal acquired from the pulse wave sensor 211, besides components due to a heartbeat, components due to a body motion and the like are also included. The same applies to a pulse wave detection signal (a pulse wave sensor signal after DC component cut) used for calculation of pulsation information. Among the components, components useful for calculation of pulsation information are the components due to the heartbeat. The components due to the body motion and the like are hindrance to calculation. Therefore, a signal due to the body motion (a body motion detection signal) is acquired using a body motion sensor. A signal component correlated to the body motion detection signal (referred to as estimatedbodymotionnoise component) is removed from the pulse wave detection signal to reduce a body motion noise component included in the pulse wave detection signal. However, even if the body motion noise component in the pulse wave detection signal and the body motion detection signal from the body motion sensor are signals due to the same body motion, signal levels thereof are not always the same. Therefore, for example, filter processing, a filter coefficient of which is adaptively determined with respect to the body motion detection signal, is performed to calculate an estimated body motion noise component and a difference between the pulse wave detection signal and a signal including only the calculated estimated body motion noise component is calculated.

Consequently, it is possible to, for example, reduce noise included in the first detection signal and improve the side lobe characteristic after the Fourier transform.

The signal processing such as the noise reduction processing can also be performed in the first embodiment. If noise can be reduced by the noise reduction processing, the signal state in the first embodiment changes. Therefore, it is possible to, for example, perform the frequency resolution processing with more importance placed on the real time property.

6. Other application examples

The signal analyzing device (the pulsation detecting device) 100 can be used not only in the pulse measurement but also in other kinds of measurement (analysis). For example, the signal analyzing device (the pulsation detecting device) 100 can be used in all fields as long as, in the fields, various sensor signals are measured and a cycle, speed, a frequency, and the like are calculated on the basis of a frequency resolution result of the sensor signals. Consequently, it is possible to calculate a cycle, speed, a frequency, and the like with an improved real time property while maintaining resolution equivalent to the resolution of the method in the past.

More specifically, the signal analyzing device 100 can be used in pitch measurement for measuring the number of steps per unit time, repetition cycle measurement for an exercise based on an acceleration detection signal, and the like. Besides, the signal analyzing device 100 can be used in a large number of kinds of measurement and analyses such as a heartbeat fluctuation analysis (an autonomic nerve analysis) for performing a frequency analysis of fluctuation in a heartbeat interval to estimate functions of an sympathetic nerve and a parasympathetic nerve, a respiratory cycle analysis, a blood pressure adjustment cycle analysis, measurement of spontaneous electric potential included in brain waves, a climatic variation analysis, and a cycle analysis of sunspot activity.

A part or most of the processing of the signal analyzing device (the pulsation detecting device) 100 in the first embodiment, the second embodiment, or the like may be realized by a program.

Consequently, it is possible to realize the processing in the first embodiment, the second embodiment, or the like with the program. The program may be, for example, a program read out and executed by a processing unit (e.g., a DSP) or the like of a device such as a smart phone.

A pulse wave detection device worn by the user may be configured by the pulse wave sensor 211 and a communication unit that communicates a pulse wave sensor signal from the pulse wave sensor 211 by radio or wire. In that case, the program is read out and executed by a processing unit (e.g., a CPU) or the like of an information processing system that is provided separately from the pulse wave detection device and receives the pulse wave sensor signal from the communication unit as explained above. The information processing system may be an information processing system not assumed to be worn by the user such as a PC or may be an information processing system assumed to be worn (carried) by the user such as a smart phone. A server system or the like connected via a network such as the Internet may be used as the information processing system.

When the pulse wave detection device and the information processing system in which the program is executed are separate, the display unit 70 used for presentation of the pulsation information to the user is provided in any place. For example, the pulsation information may be displayed on the display unit 70 of the information processing system. Alternatively, the display unit 70 may be provided in the pulse wave detection device to display the pulsation information output from the information processing system on the display unit 70. The pulsation information may be displayed on the display unit 70 of a different apparatus (e.g., any client apparatus when a server system is used as the information processing system).

The program is recorded in an information storage medium. As the information recording medium, various recording media readable by the information processing system or the like including optical disks such as a DVD and a CD, a magneto-optical disk, a hard disk (HDD), and memories such as a nonvolatile memory and a RAM can be assumed.

The first embodiment and the second embodiment are explained in detail above. However, those skilled in the art could easily understand that many modifications not substantially departing from the new matters and the effects of the present invention are possible. Therefore, all such modifications are assumed to be included in the scope of the present invention. For example, in the specification and the drawings, terms described together with broader or synonymous different terms at least once can be replaced with the different terms in all parts of the specification and the drawings. The configurations and the operations of the signal analyzing device (the pulsation detecting device) 100, the electronic apparatus, the program, and the like are not limited to those explained in the first embodiment and the second embodiment. Various modifications of the configurations and the operations are possible.

Reference Signs List

70      Display unit
100     Signal analyzing device (pulsation detecting device)
110     Signal acquiring unit
130     Signal processing unit
131     Noise-reduction processing unit
133     Window-function processing unit
135     Fourier-transform processing unit
137     Power-spectral-density-estimation processing unit
150     Signal-state determining unit
151     Spectral-value calculating unit
153     Exercise-state determining unit
170     Pulsation-information calculating unit
200     Detecting unit
210     Pulse-wave detecting unit
211     Pulse wave sensor
215     Filter processing unit
216     A/D conversion unit
220     Body-motion detecting unit
221     Body motion sensor (motion sensor)
225     Filter processing unit
226     A/D conversion unit
300     Retaining mechanism
302     Guide
400     Base unit

**Claims**

1. A pulsation detecting device comprising:

    a signal processing unit that performs frequency resolution processing on the basis of a pulse wave detection signal from a pulse-wave detecting unit and a body motion detection signal from a body-motion detecting unit;
    a pulsation- information calculating unit that calculates pulsation information on the basis of a result of the frequency resolution processing; and
    a signal-state determining unit that applies determination processing for a signal state to at least one of the pulse wave detection signal and the body motion detection signal, wherein

the signal processing unit performs a different kind of the frequency resolution processing according to a determination result of the signal state by the signal-state determining unit.

2. The pulsation detecting device according to claim 1, wherein the signal processing unit performs first frequency resolution processing when it is determined that the pulse wave detection signal is in a first signal state and performs second frequency resolution processing when it is determined that the pulse wave detection signal is in a second signal state in which an SN ratio (signal-noise ratio) is higher than an SN ratio in the first signal state.

3. The pulsation detecting device according to claim 2, wherein the signal processing unit performs window function processing for sampling data of the pulse wave detection signal and Fourier transform processing for data after the window function processing as the first frequency resolution processing when it is determined that the pulse wave detection signal is in the first signal state and performs power spectral density estimation processing as the second frequency resolution processing when it is determined that the pulse wave detection signal is in the second signal state.

4. The pulsation detecting device according to claim 3, wherein
jth sampling data $D_j$ among sampling data $D_0$ to $D_N$ (N is a positive integer equal to or larger than 2) of the pulse wave detection signal is data sampled at timing later than timing when ith sampling data $D_i$ is sampled (integers i and j are integers satisfying $0 \leq i < N/2 < j \leq N$), and
the signal processing unit performs the window function processing using an asymmetrical window function, a window function value $h(j)$ of which corresponding to the jth sampling data $D_j$ is a maximum and a window function value $h(i)$ of which corresponding to the ith sampling data $D_i$ is a value smaller than the window function value $h(j)$, when it is determined that the pulse wave detection signal is in the first signal state.

5. The pulsation detecting device according to claim 2, wherein the signal processing unit performs window function processing for sampling data of the pulse wave detection signal, in which a first window function is used, and Fourier transform processing for first data after the window function processing as the first frequency resolution processing when it is determined that the pulse wave detection signal is in the first signal state and performs the window function processing for the sampling data of the pulse wave detection signal, in which a second window function different from the first window function is used, and the Fourier transform processing for second data after the window function processing as the second frequency resolution processing when it is determined that the pulse wave detection signal is in the second signal state.

6. The pulsation detecting device according to claim 5, wherein
jth sampling data $D_j$ among sampling data $D_0$ to $D_N$ (N is a positive integer equal to or larger than 2) of the pulse wave detection signal is data sampled at timing later than timing when ith sampling data $D_i$ is sampled (integers i and j are integers satisfying $0 \leq i < N/2 < j \leq N$), and
the signal processing unit performs the window function processing using an asymmetrical window function, a window function value $h(j)$ of which corresponding to the jth sampling data $D_j$ is a maximum and a window function value $h(i)$ of which corresponding to the ith sampling data $D_i$ is a value smaller than the window function value $h(j)$, as the first window function when it is determined that the pulse wave detection signal is in the first signal state and performs the window function processing using a second window function, which is the asymmetrical window function different from the first window function, when it is determined that the pulse wave detection signal is in the second signal state.

7. The pulsation detecting device according to any one of claims 1 to 6, wherein the signal-state determining unit calculates a ratio of spectrum values of a first spectrum indicating a maximum spectrum value and at least one second spectrum other than the first spectrum among frequency spectra obtained by the frequency resolution processing of the pulse wave detection signal and performs the determination processing for the signal state.

8. The pulsation detecting device according to claim 1, wherein
the signal-state determining unit performs processing for determining an exercise state of a subject on the basis of the body motion detection signal as the determination processing for the signal state, and
the signal processing unit performs a different kind of the frequency resolution processing according to the exercise state.

9. The pulsation detecting device according to claim 8, wherein the signal processing unit performs first frequency resolution processing when it is determined that the exercise state is a steady exercise state and performs second frequency resolution processing when it is determined that the exercise state is an unsteady exercise state.

10. The pulsation detecting device according to claim 9, wherein the signal processing unit performs window function processing for sampling data of the pulse wave detection signal, in which a first window function is used, and Fourier transform processing for first data after the window function processing as the first frequency resolution processing when it is determined that the exercise state is the steady exercise state and performs the window function processing for the sampling data of the pulse wave detection signal, in which a second window function different from the first window function is used, and the Fourier transform process ing for second data after the window function processing as the second frequency resolution processing when it is determined that the exercise state is the unsteady exercise state.

11. The pulsation detecting device according to claim 10, wherein
jth sampling data $D_j$ among sampling data $D_0$ to $D_N$ (N is a positive integer equal to or larger than 2) of the pulse wave detection signal is data sampled at timing later than timing when ith sampling data $D_i$ is sampled (integers i and j are integers satisfying $0 \leq i < N/2 < j \leq N$), and
the signal processing unit performs the window function processing using an asymmetrical window function, a window function value $h(j)$ of which corresponding to the jth sampling data $D_j$ is a maximum and a window function value $h(i)$ of which corresponding to the ith sampling data $D_i$ is a value smaller than the window function value $h(j)$, as the first window function when it is determined that the exercise state is the steady exercise state and performs the window function processing using a second window function, which is the asymmetrical window function different from the first window function, when it is determined that the exercise state is the unsteady exercise state.

12. The pulsation detecting device according to claim 8, wherein the signal processing unit performs first frequency resolution processing when it is determined that the exercise state is a rest state and performs second frequency resolution processing when it is determined that the exercise state is a steady exercise state or an unsteady exercise state.

13. The pulsation detecting device according to claim 12, wherein the signal processing unit performs power spectral density estimation processing as the first frequency resolution processing when it is determined that the exercise state is the rest state and performs window function processing for sampling data of the pulse wave detection signal and Fourier transform processing for data after the window function processing as the second frequency resolution processing when it is determined that the exercise state is the in-exercise state.

14. The pulsation detecting device according to claim 13, wherein
jth sampling data $D_j$ among sampling data $D_0$ to $D_N$ (N is a positive integer equal to or larger than 2) of the pulse wave detection signal is data sampled at timing later than timing when ith sampling data $D_i$ is sampled (integers i and j are integers satisfying $0 \leq i < N/2 < j \leq N$), and
the signal processing unit performs the window function processing using an asymmetrical window function, a window function value $h(j)$ of which corresponding to the jth sampling data $D_j$ is a maximum and a window function value $h(i)$ of which corresponding to the ith sampling data $D_i$ is a value smaller than the window function value $h(j)$, as the first window function when it is determined that the exercise state is the steady exercise state or the unsteady exercise state.

15. An electronic apparatus comprising the signal analyzing device according to any one of claims 1 to 14.

16. A program for causing a computer to function as:

a signal processing unit that performs frequency resolution processing on the basis of a pulse wave detection signal from a pulse-wave detecting unit and a body motion detection signal from a body-motion detecting unit;
a pulsation-information calculating unit that calculates pulsation information on the basis of a result of the frequency resolution processing; and
a signal-state determining unit that applies determination processing for a signal state to at least one of the pulse wave detection signal and the body motion detection signal, wherein
the signal processing unit performs a different kind of the frequency resolution processing according to a determination result of the signal state by the signal-state determining unit.

EP 2 832 288 A1

FIG. 1

100

300 211

PULSE
RATE 72
8:15 AM

400

70

## FIG. 2A

221

302

100

211

## FIG. 2B

FIG. 3

EP 2 832 288 A1

| TYPE OF FREQUENCY RESOLUTION PROCESSING | POWER SPECTRUM DENSITY ESTIMATION | WINDOW FUNCTION TYPE3 | WINDOW FUNCTION TYPE1 | WINDOW FUNCTION TYPE2 |
|---|---|---|---|---|
| REAL TIME PROPERTY | HIGH | ⇔ | | LOW |
| ADAPTABILITY OF PULSE WAVE DETECTION SIGNAL WITH LOW SN RATIO | LOW | ⇔ | | HIGH |
| MAGNITUDE OF SIDE LOBE INFLUENCE | — | LARGE | ⇔ | SMALL |

FIG. 4

EP 2 832 288 A1

START

S100

ACQUIRE DETECTION SIGNAL DATA — S101

DETERMINE SIGNAL STATE OF
PULSE WAVE DETECTION SIGNAL — S102

BODY MOTION REMOVAL
FILTER PROCESSING — S103

DETERMINE FREQUENCY
RESOLUTION PROCESSING OF
PULSE WAVE DETECTION SIGNAL — S104

FREQUENCY RESOLUTION
PROCESSING FOR PULSE
WAVE DETECTION SIGNAL — S105

SPECIFY PULSE FREQUENCY — S106

CONVERT PULSE FREQUENCY
INTO PULSE RATE — S107

DISPLAY PULSE RATE — S108

CONTINUE
MEASUREMENT ?     S109     false

true

S110

END

FIG. 5

```
                    ┌─────────────┐
                    │    START    │
                    └──────┬──────┘
                           ▼
        ┌──────────────────────────────────┐
        │ ACQUIRE PULSE WAVE DETECTION      │
        │ SIGNAL DATA AND BODY MOTION       │ ～S200
        │ DETECTION SIGNAL DATA EQUIVALENT  │
        │ TO 256 SAMPLES IN 16 SECONDS      │
        └──────────────────┬───────────────┘
                           ▼
        ┌──────────────────────────────────┐
        │ APPLY NORMAL FFT PROCESSING TO    │
        │ ACQUIRED PULSE WAVE DETECTION     │ ～S201
        │ SIGNAL DATA AND ACQUIRED BODY     │
        │ MOTION DETECTION SIGNAL           │
        └──────────────────┬───────────────┘
                           ▼
        ┌──────────────────────────────────┐
        │ SORT SPECTRA OF PULSE             │
        │ WAVE DETECTION SIGNAL             │ ～S202
        │ IN DESCENDING ORDER              │
        └──────────────────┬───────────────┘
                           ▼
        ┌──────────────────────────────────┐
        │ CALCULATE PULSATION SIGNAL        │ ～S203
        │ SPECTRUM RATIO pr5, pr10          │
        └──────────────────┬───────────────┘
```

S204

pr5 < 0.5 and pr10 < 0.2 ?   false

true

S206

pr5 > 0.7 and pr10 > 0.5 ?   false

true

S208

DETERMINE THAT STATE OF SN RATIO IS SATISFACTORY AND DETERMINE THAT POWER SPECTRAL DENSITY ESTIMATION PROCESSING IS PERFORMED   ～S205

DETERMINES THAT STATE OF SN RATIO IS MEDIUM AND DETERMINE THAT WINDOW FUNCTION PROCESSING OF TYPE 3 + FFT ARE PERFORMED

DETERMINE THAT STATE OF SN RATIO IS INFERIOR AND DETERMINES THAT WINDOW FUNCTION PROCESSING OF TYPE 2 + FFT ARE PERFORMED   ～S207

┌─────────────┐
│     END     │
└─────────────┘

# FIG. 6

| adr | val | adr | val |
|---|---|---|---|
| 0 | 0.000 | 32 | 368.897 |
| 1 | 40.586 | 33 | 742.109 |
| 2 | 80.571 | 34 | 987.781 |
| 3 | 73.855 | 35 | 2973.406 |
| 4 | 11.919 | 36 | 1164.529 |
| 5 | 83.755 | 37 | 905.476 |
| 6 | 23.659 | 38 | 880.062 |
| 7 | 81.123 | 39 | 221.764 |
| 8 | 54.354 | 40 | 1050.329 |
| 9 | 69.027 | 41 | 1497.683 |
| 10 | 18.965 | 42 | 1033.663 |
| 11 | 250.288 | 43 | 503.500 |
| 12 | 75.841 | 44 | 290.433 |
| 13 | 140.628 | 45 | 271.738 |
| 14 | 39.786 | 46 | 154.906 |
| 15 | 59.384 | 47 | 219.004 |
| 16 | 41.394 | 48 | 165.998 |
| 17 | 179.019 | 49 | 199.515 |
| 18 | 220.117 | 50 | 101.020 |
| 19 | 209.179 | 51 | 85.193 |
| 20 | 172.737 | 52 | 90.860 |
| 21 | 62.916 | 53 | 183.259 |
| 22 | 198.035 | 54 | 16.251 |
| 23 | 219.786 | 55 | 34.060 |
| 24 | 241.945 | 56 | 254.988 |
| 25 | 58.415 | 57 | 145.070 |
| 26 | 180.971 | 58 | 121.124 |
| 27 | 149.069 | 59 | 205.957 |
| 28 | 85.328 | 60 | 213.124 |
| 29 | 91.605 | 61 | 385.227 |
| 30 | 200.110 | 62 | 220.148 |
| 31 | 145.144 | 63 | 97.991 |

FIRST SPECTRUM
POSITION : 35

**FIG. 7A**

| sort | adr |
|---|---|
| 1 | 35 |
| 2 | 41 |
| 3 | 36 |
| 4 | 40 |
| 5 | 42 |
| 6 | 34 |
| 7 | 37 |
| 8 | 38 |
| 9 | 33 |
| 10 | 43 |
| 11 | 61 |
| 12 | 32 |
| 13 | 44 |
| 14 | 45 |
| 15 | 56 |
| 16 | 11 |
| 17 | 24 |
| 18 | 39 |
| 19 | 62 |
| 20 | 18 |
| 21 | 23 |
| 22 | 47 |
| 23 | 60 |
| 24 | 19 |
| 25 | 59 |
| 26 | 30 |
| 27 | 49 |
| 28 | 22 |
| 29 | 53 |
| 30 | 26 |
| 31 | 17 |
| 32 | 20 |

FIFTH SPECTRUM
POSITION : 42

**FIG. 7B**

TENTH SPECTRUM
POSITION : 43

**FIG. 7C**

| adr | val | adr | val |
|---|---|---|---|
| 0 | 0.000 | 32 | 239.897 |
| 1 | 8.595 | 33 | 995.954 |
| 2 | 37.068 | 34 | 769.779 |
| 3 | 95.847 | 35 | 184.164 |
| 4 | 138.272 | 36 | 553.503 |
| 5 | 133.412 | 37 | 46.414 |
| 6 | 138.461 | 38 | 1682.961 |
| 7 | 290.188 | 39 | 966.913 |
| 8 | 336.633 | 40 | 159.421 |
| 9 | 184.753 | 41 | 848.048 |
| 10 | 322.254 | 42 | 2674.049 |
| 11 | 207.133 | 43 | 192.952 |
| 12 | 523.722 | 44 | 745.337 |
| 13 | 988.846 | 45 | 628.515 |
| 14 | 1612.317 | 46 | 930.617 |
| 15 | 1152.304 | 47 | 428.586 |
| 16 | 1115.757 | 48 | 196.617 |
| 17 | 442.341 | 49 | 317.008 |
| 18 | 933.821 | 50 | 567.846 |
| 19 | 1102.984 | 51 | 560.959 |
| 20 | 995.690 | 52 | 369.451 |
| 21 | 380.485 | 53 | 640.457 |
| 22 | 230.177 | 54 | 740.112 |
| 23 | 998.515 | 55 | 731.483 |
| 24 | 1189.295 | 56 | 660.948 |
| 25 | 1387.373 | 57 | 548.250 |
| 26 | 1431.684 | 58 | 433.319 |
| 27 | 971.278 | 59 | 424.290 |
| 28 | 1367.759 | 60 | 231.912 |
| 29 | 653.904 | 61 | 118.322 |
| 30 | 734.653 | 62 | 267.317 |
| 31 | 137.883 | 63 | 382.830 |

| sort | adr |
|---|---|
| 1 | 42 |
| 2 | 38 |
| 3 | 14 |
| 4 | 26 |
| 5 | 25 |
| 6 | 28 |
| 7 | 24 |
| 8 | 15 |
| 9 | 16 |
| 10 | 19 |
| 11 | 23 |
| 12 | 33 |
| 13 | 20 |
| 14 | 13 |
| 15 | 27 |
| 16 | 39 |
| 17 | 18 |
| 18 | 46 |
| 19 | 41 |
| 20 | 34 |
| 21 | 44 |
| 22 | 54 |
| 23 | 30 |
| 24 | 55 |
| 25 | 56 |
| 26 | 29 |
| 27 | 53 |
| 28 | 45 |
| 29 | 50 |
| 30 | 51 |
| 31 | 36 |
| 32 | 57 |

FIRST SPECTRUM
POSITION : 42

FIFTH SPECTRUM
POSITION : 25

TENTH SPECTRUM
POSITION : 19

FIG. 8A    FIG. 8B    FIG. 8C

| adr | val | adr | val |
|---|---|---|---|
| 0 |  | 32 | 1216.799 |
| 1 | 259.639 | 33 | 1020.420 |
| 2 | 715.738 | 34 | 826.489 |
| 3 | 1038.658 | 35 | 601.463 |
| 4 | 1368.646 | 36 | 336.918 |
| 5 | 2143.715 | 37 | 311.661 |
| 6 | 657.774 | 38 | 643.498 |
| 7 | 1873.973 | 39 | 767.436 |
| 8 | 1815.470 | 40 | 381.051 |
| 9 | 2178.379 | 41 | 285.870 |
| 10 | 622.735 | 42 | 199.695 |
| 11 | 1431.467 | 43 | 346.537 |
| 12 | 1855.991 | 44 | 361.788 |
| 13 | 1061.363 | 45 | 245.467 |
| 14 | 1825.265 | 46 | 430.311 |
| 15 | 1132.039 | 47 | 348.549 |
| 16 | 65.312 | 48 | 571.318 |
| 17 | 259.574 | 49 | 302.138 |
| 18 | 2058.390 | 50 | 452.714 |
| 19 | 711.162 | 51 | 397.176 |
| 20 | 1701.423 | 52 | 428.871 |
| 21 | 922.353 | 53 | 331.839 |
| 22 | 1015.190 | 54 | 543.090 |
| 23 | 1587.461 | 55 | 203.054 |
| 24 | 840.735 | 56 | 697.760 |
| 25 | 1275.321 | 57 | 306.664 |
| 26 | 1345.791 | 58 | 177.090 |
| 27 | 1380.851 | 59 | 356.382 |
| 28 | 1779.537 | 60 | 132.409 |
| 29 | 1417.881 | 61 | 252.088 |
| 30 | 929.004 | 62 | 27.344 |
| 31 | 735.065 | 63 | 344.399 |

FIRST SPECTRUM
POSITION : 9

**FIG. 9A**

| sort | adr |
|---|---|
| 1 | 9 |
| 2 | 5 |
| 3 | 18 |
| 4 | 7 |
| 5 | 12 |
| 6 | 14 |
| 7 | 8 |
| 8 | 28 |
| 9 | 20 |
| 10 | 23 |
| 11 | 11 |
| 12 | 29 |
| 13 | 27 |
| 14 | 4 |
| 15 | 26 |
| 16 | 25 |
| 17 | 32 |
| 18 | 15 |
| 19 | 13 |
| 20 | 3 |
| 21 | 33 |
| 22 | 22 |
| 23 | 30 |
| 24 | 21 |
| 25 | 24 |
| 26 | 34 |
| 27 | 39 |
| 28 | 31 |
| 29 | 2 |
| 30 | 19 |
| 31 | 56 |
| 32 | 6 |

FIFTH SPECTRUM
POSITION : 12

**FIG. 9B**

PULSE WAVE DETECTION SIGNAL

FFT RESULT

16 (SEC-ONDS)

4[Hz]

TENTH SPECTRUM
POSITION : 23

**FIG. 9C**

33

FIG.10A

PULSE WAVE DETECTION SIGNAL

0        16(SECONDS)

FFT RESULT

0        4[Hz]

FIG.10B

PULSE WAVE DETECTION SIGNAL

0        16(SECONDS)

FFT RESULT

0        4[Hz]

FIG.10C

PULSE WAVE DETECTION SIGNAL

0        16(SECONDS)

FFT RESULT

0        4[Hz]

START

S300

ACQUIRE DETECTION SIGNAL DATA — S301

DETERMINE EXERCISE STATE — S302

BODY MOTION NOISE REMOVAL FILTER PROCESSING — S303

DETERMINE FREQUENCY RESOLUTION PROCESSING OF PULSE WAVE DETECTION SIGNAL — S304

FREQUENCY RESOLUTION PROCESSING OF PULSE WAVE DETECTION SIGNAL — S305

SPECIFY PULSE FREQUENCY — S306

CONVERT PULSE FREQUENCY INTO PULSE RATE — S307

DISPLAY PULSE RATE — S308

CONTINUE MEASUREMENT ? S309 — false

true

S310

END

FIG.11

FIG.12

FIG.13

EP 2 832 288 A1

FIG.14A

FIG.14B

FIG.14C

**FIG.15A**

**FIG.15B**

FIG.16A

FIG.16B

## INTERNATIONAL SEARCH REPORT

International application No.

PCT/JP2013/002066

**A. CLASSIFICATION OF SUBJECT MATTER**
*A61B5/0245*(2006.01)i, *A61B5/11*(2006.01)i

According to International Patent Classification (IPC) or to both national classification and IPC

**B. FIELDS SEARCHED**

Minimum documentation searched (classification system followed by classification symbols)
A61B5/0245, A61B5/11

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2013 |
| Kokai Jitsuyo Shinan Koho | 1971-2013 | Toroku Jitsuyo Shinan Koho | 1994-2013 |

Electronic data base consulted during the international search (name of data base and, where practicable, search terms used)
CiNii

**C. DOCUMENTS CONSIDERED TO BE RELEVANT**

| Category* | Citation of document, with indication, where appropriate, of the relevant passages | Relevant to claim No. |
|---|---|---|
| A | JP 2002-17694 A (Denso Corp., Akira IWATA), 22 January 2002 (22.01.2002), paragraphs [0010] to [0029]; fig. 1 to 7 (Family: none) | 1-16 |
| A | WO 2012/086175 A1 (Sony Computer Entertainment Inc.), 28 June 2012 (28.06.2012), paragraphs [0018] to [0035]; fig. 2 & JP 2012-086175 A | 1-16 |

☒ Further documents are listed in the continuation of Box C.   ☐ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 17 April, 2013 (17.04.13) | 07 May, 2013 (07.05.13) |

| Name and mailing address of the ISA/ | Authorized officer |
|---|---|
| Japanese Patent Office | |
| Facsimile No. | Telephone No. |

Form PCT/ISA/210 (second sheet) (July 2009)

**INTERNATIONAL SEARCH REPORT**

| International application No. |
|---|
| PCT/JP2013/002066 |

C (Continuation).   DOCUMENTS CONSIDERED TO BE RELEVANT

| Category* | Citation of document, with indication, where appropriate, of the relevant passages | Relevant to claim No. |
|---|---|---|
| A | WO 99/26529 A1  (Seiko Epson Corp.),<br>03 June 1999 (03.06.1999),<br>description, page 27, line 4 to page 29, line 21; fig. 1<br>& US 6293915 B1          & EP 968681 A1<br>& DE 69832782 D          & CN 1251027 A<br>& HK 1027496 A          & TW 425278 B | 1-16 |

Form PCT/ISA/210 (continuation of second sheet) (July 2009)

**EP 2 832 288 A1**

**Patent documents cited in the description**

- JP 2007054471 A